(19) Europäisches Patentamt
European Patent Office
Office européen des brevets

(11) **EP 3 113 033 B1**

(12) **FASCICULE DE BREVET EUROPEEN**

(45) Date de publication et mention de la délivrance du brevet:
**09.01.2019 Bulletin 2019/02**

(51) Int Cl.:
*A61N 1/36* (2006.01)          *A61N 1/05* (2006.01)
*G06F 17/16* (2006.01)          *G06F 19/00* (2018.01)

(21) Numéro de dépôt: **16173737.4**

(22) Date de dépôt: **09.06.2016**

(54) **SYSTÈME DE TRAITEMENT PAR STIMULATION, NOTAMMENT DU NERF VAGUE, PAR MISE EN OEUVRE D'UN MODÈLE DE TRANSITION D'ÉTATS**

STIMULATIONSBEHANDLUNGSSYSTEM, INSBESONDERE FÜR DEN VAGUSNERV DURCH ANWENDUNG EINES ZUSTANDSÜBERGANGSMODELLS

STIMULATION THERAPY SYSTEM, IN PARTICULAR OF THE VAGUS NERVE, BY IMPLEMENTING A STATE TRANSITION MODEL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.06.2015 FR 1556045**

(43) Date de publication de la demande:
**04.01.2017 Bulletin 2017/01**

(73) Titulaires:
• **Sorin CRM SAS**
  **92140 Clamart Cedex (FR)**
• **Université de Rennes 1**
  **35065 Rennes Cedex (FR)**
• **INSERM - Institut National de la Santé et de la Recherche Médicale**
  **75013 Paris (FR)**

(72) Inventeurs:
• **BONNET, Jean-Luc**
  **91300 MASSY (FR)**
• **HERNANDEZ, Alfredo**
  **35510 Cesson Sévigné (FR)**
• **ROMERO UGALDE, Hector Manuel**
  **35000 Rennes (FR)**
• **LE ROLLE, Virginie**
  **35510 Cesson-Sévigné (FR)**
• **CARRAULT, Guy**
  **35510 Cesson-Sévigné (FR)**

(74) Mandataire: **Grünecker Patent- und Rechtsanwälte PartG mbB Leopoldstraße 4 80802 München (DE)**

(56) Documents cités:
EP-A2- 1 102 607          US-A1- 2005 240 242
US-A1- 2006 293 720       US-A1- 2009 326 624
US-A1- 2010 241 020       US-A1- 2013 274 625
US-A1- 2013 317 580       US-A1- 2014 288 620

**Description**

L'invention concerne d'une façon générale les dispositifs destinés à appliquer sur des organes une stimulation à des fins thérapeutiques.

Elle est notamment applicable aux " dispositifs médicaux implantables actifs" tels que définis par la directive 90/385/CEE du 20 juin 1990 du Conseil des communautés européennes . Cette définition inclut notamment les appareils chargés de surveiller en continu l'activité cardiaque et délivrer si nécessaire au coeur des impulsions électriques de stimulation, de resynchronisation et/ou de défibrillation en cas de trouble du rythme détecté par le dispositif. Elle inclut aussi les appareils neurologiques, les implants cochléaires, etc., ainsi que les dispositifs de mesure de pH ou autres paramètres intracorporels.

Quelle que soit la pathologie à traiter, une thérapie de stimulation vise en général à maximiser les effets thérapeutiques, tout en minimisant les effets secondaires, ainsi que la consommation d'énergie dans le cas où la stimulation est mise en oeuvre dans un dispositif implantable autonome. Cette stimulation doit en même temps tenir compte de la dynamique de la pathologie résultant par exemple d'une altération du système nerveux autonome (ANS, Autonomic Nervous System), d'un remodelage cardiaque ou de l'ANS, ainsi que de la réponse à la thérapie (habituation, modification du couplage électrode-nerf vague dans le cas de la stimulation du nerf vague (VNS, Vagus Nerve Stimulation).

Ainsi l'application d'une stimulation optimale est un problème complexe, imparfaitement traité à l'heure actuelle bien que représentant l'une des priorités les plus hautes dans les progrès de la neurostimulation.

Il existe à l'heure actuelle des approches de commande de la stimulation en boucle fermée, telle que celles décrites dans les US 2014/288620 A1, US 2006/293720 A1 ou US 2005/240242 A1, qui peuvent être classées en deux familles :

1) les approches à base de règles, telles que celles décrites dans les US 7 783 349 B2 , US 7 509 166 B2 , US 2012/245656 A1 ou encore WO 2011/137029 A ;

2) les approches à base d'une fonction de transfert linéaire ou non linéaire des variables de contrôle, telles que celles décrites par exemple dans le EP 1 102 607 A1 .

La première approche a deux limites majeures : i) il est difficile de définir des règles optimales pour un patient donné et ii) ces règles reposent sur la définition de seuils, qui peuvent varier en fonction de la variabilité inter- et intra-patient.

Les principales limites de la deuxième approche sont quant à elles i) la complexité de calcul nécessaire pour mettre en oeuvre les dispositifs de commande, et ii) le grand nombre de données nécessaires pour ajuster les paramètres du dispositif de commande.

Il existe par ailleurs d'autres méthodes pour estimer un ensemble de règles spécifiques aux patients [1,2], d'inférer des ajustements inter- ou intra-patients [3], et pour réduire la complexité des calculs à effectuer dans le dispositif de commande [4, 5]. Ces approches restent toutefois théoriques et difficiles à mettre en oeuvre et à intégrer dans les dispositifs implantables actifs, dotés d'une puissance de traitement numérique limitée. Les références pertinentes sont :

[1] A. Aarabi, and H. Bin, "Seizure prédiction in intracranial EEG: A patient-specific rule-based approach", Engineering in Medicine and Biology Society, EMBC, 2011 Annual International Conference of the IEEE, 2566 - 2569, Boston, MA ;

[2] I. Capel, M. Rigla, G. García-Sáez, A. Rodriguez-Herrero, B. Pons, D. Subías, F. García-García, M. Gallach, M. Aguilar, C. Pérez-Gandía, E. J. Gómez, A. Caixàs, and M. E. Hernando, "Artificial Pancreas Using a Personalized Rule-Based Controller Achieves Overnight Normoglycemia in Patients with Type 1 Diabetes", Diabetes Technol Ther., Vol. 16(3): 172-179 (2014) ;

[3] F. Porée, A. Kachenoura, G. Carrault, R. D. Molin, P. Mabo, and A. I. Hernández, "Surface Electrocardiogram Reconstruction From Intra-cardiac Electrograms Using a Dynamic Time Delay Artificial Neural Network", Biomedical Engineering, IEEE Transactions on, Vol. 60, 106-114, (2013).

[4] H. M. Romero Ugalde, J.-C. Carmona, V. M. Alvarado and J. Reyes-Reyes, "Neural Network Design and Model Reduction Approach for Black Box Non Linear System Identification with Reduced Number of Parameters", Neurocomputing, Vol. 101, 170-180 (2013).

[5] M. Lôhning, M. Reble, J. Hasenauer, S. Yu, F. Allgöwer, "Model predictive control using reduced order models: Guaranteed stability for constrained linear systems", Journal of Process Control, Vol. 24 (11), 1647-1659, (2014).

Enfin, une autre limitation majeure des approches existantes concerne la constante de temps de la commande en boucle fermée, qui est généralement figée sur une unique échelle de temps prédéfinie (par exemple à chaque battement cardiaque, chaque minute, chaque jour, etc.), en particulier dans le cas où les variables commandées sont les résultats des processus qui s'entremêlent sur différentes échelles temporelles, comme en physiologie.

La présente invention vise à pallier ces limitations de l'état de la technique et à proposer une commande de stimulation qui ne nécessite que des moyens de calcul limités, tout en étant extrêmement souple et apte à ajuster de façon très fine la stimulation à la situation physique et/ou physiologique observée.

Plus précisément, l'invention propose à cet effet un système de traitement par stimulation, comprenant :

- un dispositif de stimulation tel qu'un générateur d'impulsions connecté à une ou plusieurs électrodes aptes à être placées sur ou à proximité d'un nerf du système nerveux autonome ;
- au moins un capteur de signaux physiologiques et/ou physiques et des unités de traitement de ces signaux, capables de fournir au moins un niveau physiologique/physique courant ; et
- des moyens de commande du dispositif de stimulation selon un jeu de paramètres de stimulation.

De façon caractéristique de l'invention, ce système comprend au moins un module de contrôle comportant :

- un modèle de transition d'états, ledit modèle comprenant une matrice de transition et une matrice de connexion, les états dudit modèle étant caractérisés chacun par un jeu de valeurs des paramètres de stimulation, associé à au moins une réponse physiologique/physique attendue lors de l'application d'une stimulation avec ces paramètres ; et
- un dispositif de commande de transition d'états déterminant de façon organisée les transitions d'un état courant vers un nouvel état, provoquant un changement correspondant du jeu de valeurs des paramètres utilisés pour la stimulation, d'un jeu de paramètres courant (Pi) vers un nouveau jeu de paramètres.

Dans une première forme de mise en oeuvre, le modèle de transition d'états est de type déterministe, les matrices de transition étant connues à l'initialisation du système.

Selon diverses caractéristiques subsidiaires avantageuses :

- le dispositif de commande est apte à détecter une situation physique et/ou physiologique anormale et à réaliser une transition vers un état d'absence de stimulation en réponse à cette détection ;
- le dispositif de commande est apte à déterminer d'éventuelles transitions entre états de façon régulière et prédéterminée dans le temps ;
- le dispositif de commande est apte à déterminer d'éventuelles transitions entre états en réponse à la survenue d'un ou plusieurs évènements prédéterminés ;
- le modèle de transition déterministe est séquentiel, les différents états étant ordonnés selon l'effet qu'ils provoquent sur le patient, une transition à partir d'un état ne pouvant être effectuée qu'avec un pas donné, fixe ou variable, vers un état d'ordre supérieur, vers un état d'ordre inférieur, ou vers le même état ;
- le modèle de transition déterministe est à base de dichotomie entre états de niveau minimal et de niveau maximal ;
- le modèle de transition déterministe est optimisé, le dispositif de commande déterminant l'état contenant la réponse la plus basse pour laquelle la différence entre le niveau physiologique mesuré et le niveau physiologique cible est inférieure à un seuil donné ;

Dans une seconde forme de mise en oeuvre, la ou les matrices mémorisées correspondent à un modèle de transition stochastique, chaque cellule contenant une valeur de probabilité de transition d'un état initial vers un nouvel état, et la somme des valeurs de probabilités des transitions possibles à partir d'un état donné vers tout nouvel état étant égale à 1. Selon diverses caractéristiques subsidiaires avantageuses :

- le modèle de transition d'état est un Modèle de Markov Caché (*Hidden Markov Model*);
- le dispositif de commande est apte à détecter une situation physique et/ou physiologique anormale et à réaliser une transition vers un état d'absence de stimulation en réponse à cette détection.

Dans tous les cas, le au moins un capteur est de préférence un capteur apte à être intégré dans un dispositif médical implantable, et apte à délivrer: un signal d'électrogramme cardiaque, musculaire ou nerveux ; un signal d'accélération corporelle, cardiaque ou musculaire ; un signal de bioimpédance respiratoire, de débit cardiaque ou de pression ; un signal de température ; et/ou un signal piézométrique de pression ou de contractilité cardiaque.

Le niveau physiologique et physique peut être notamment déterminé à partir de l'une des variables physiologiques ou physiques suivantes : fréquence cardiaque, variabilité du rythme sinusal, pression artérielle, contractilité cardiaque, activité physique, température, mouvements, fréquence respiratoire, ou toute combinaison de ces variables.

On va maintenant décrire un exemple de mise en oeuvre de la présente invention, en référence aux dessins annexés où les mêmes références désignent d'une Figure à l'autre des éléments identiques ou fonctionnellement semblables.

La Figure 1 illustre schématiquement l'architecture d'un système de stimulation à boucle fermée auquel s'applique l'invention.

La Figure 2 représente plus en détail l'architecture du système de commande à transition d'état selon l'invention.

La Figure 3 illustre encore plus en détail ce système et un modèle particulier de transition d'état.

La Figure 4 illustre différents algorithmes pouvant être mis en oeuvre dans un dispositif de commande de transition d'état.

La Figure 5 illustre graphiquement un mode particulier de transition entre états.

La Figure 6 illustre l'architecture d'un dispositif de commande de transition d'état déterministe et séquentiel à pas fixe.

La Figure 7 illustre les changements d'état courant en réponse à l'action de ce dispositif de commande.

La Figure 8 illustre un autre mode de changement d'état courant et l'allure correspondante d'un niveau physiologique réel par rapport à un niveau physiologique cible.

La Figure 9 illustre l'architecture d'un dispositif de commande de transition d'état déterministe et séquentiel à pas variable.

La Figure 10 illustre l'architecture d'un dispositif de commande de transition d'état déterministe à base dichotomique.

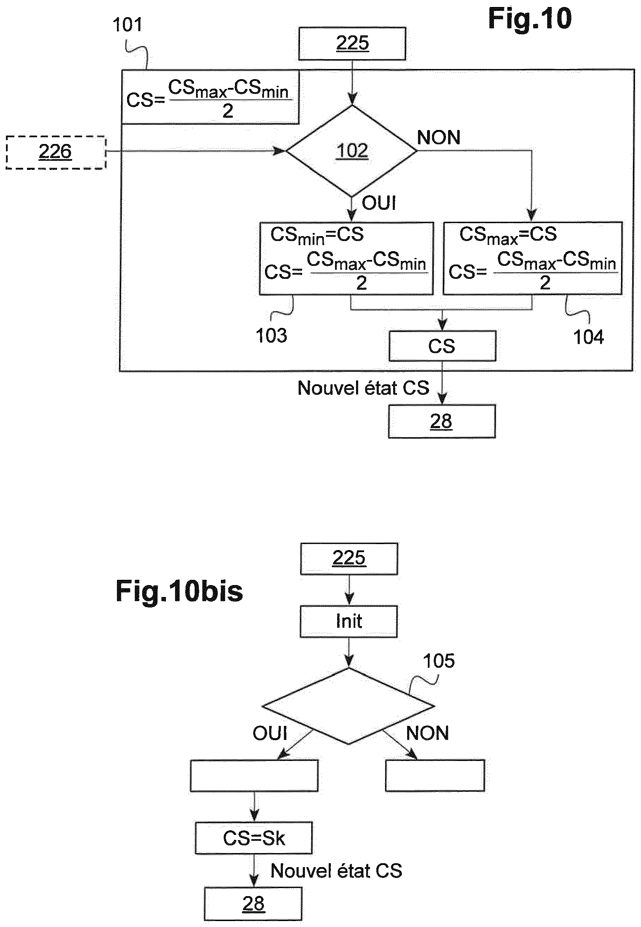

La Figure 10bis illustre l'architecture d'un dispositif de commande de transition d'état déterministe optimisé.

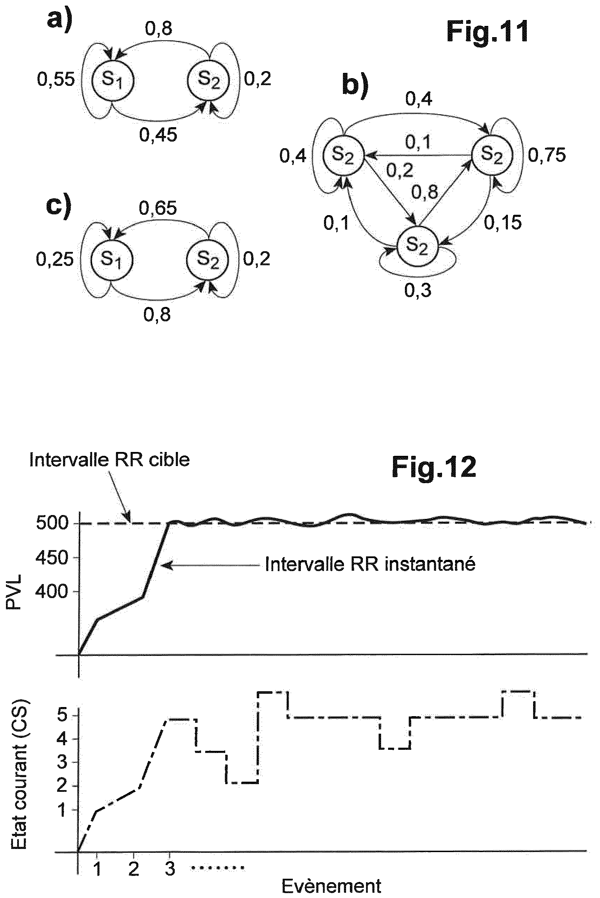

La Figure 11 illustre des transitions entre états contrôlées par un modèle de transition stochastique.

La Figure 12 représente l'évolution des états et l'évolution du niveau physiologique réel par rapport à un niveau physiologique cible avec ce modèle stochastique.

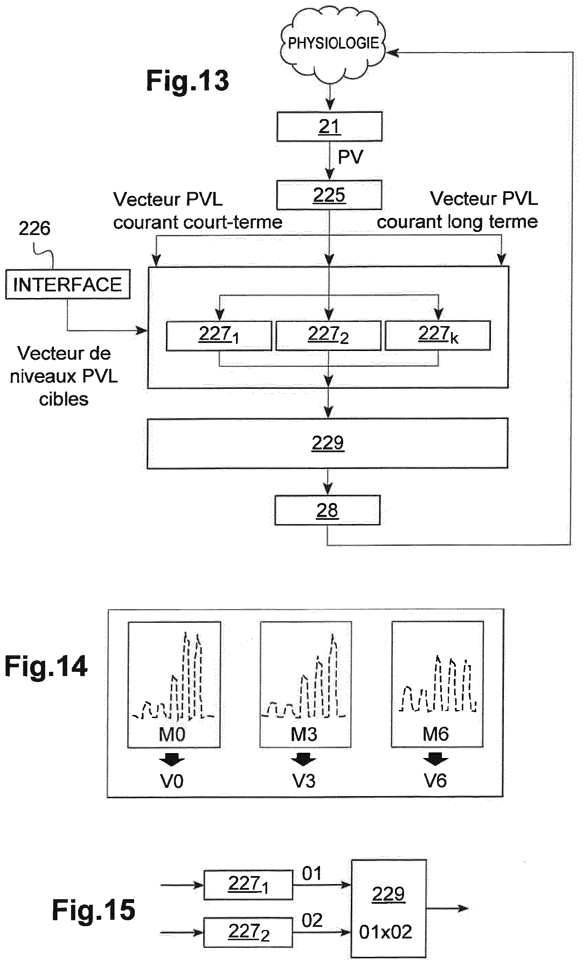

La Figure 13 illustre l'architecture d'un dispositif de commande de transition d'état à résolutions temporelles multiples.

La Figure 14 illustre différentes réponses à une même stimulation en réponse à des évolutions physiologiques de long terme, à des instants espacés également sur le long terme.

La Figure 15 illustre une combinaison (ou fusion) de consignes de transition d'état délivrées par deux dispositifs de commande de transition d'état différents d'un même système.

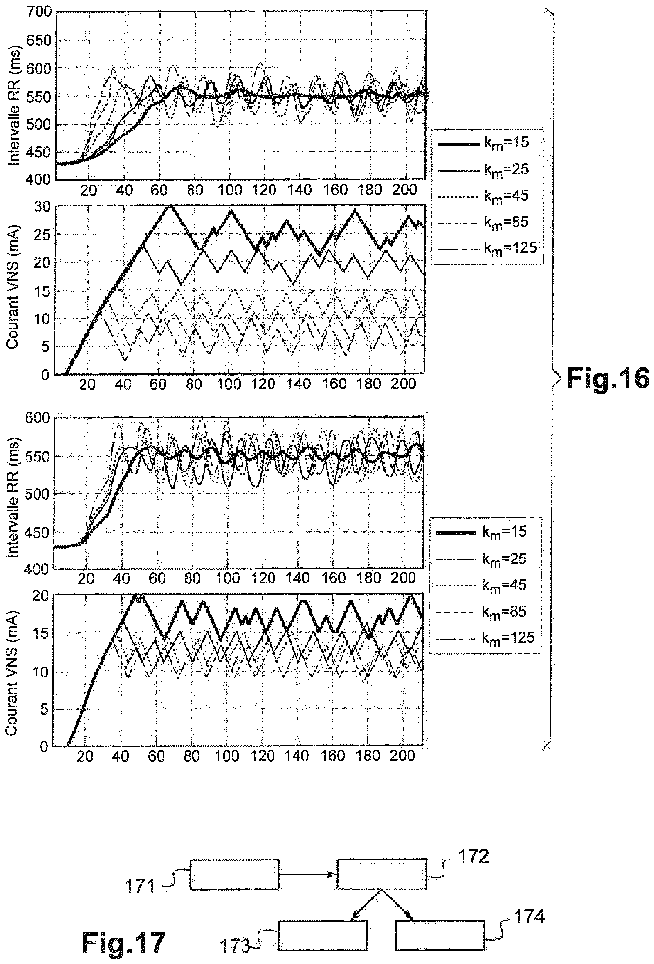

La Figure 16 illustre comparativement les comportements des réponses à des changements d'états respectivement avec un seul dispositif de commande de transition d'état sur le court terme et avec deux dispositifs de commande de transition d'état respectivement sur le court terme et sur le long terme.

La Figure 17 illustre différentes possibilités d'un mécanisme d'apprentissage pour un dispositif de commande à transition d'état.

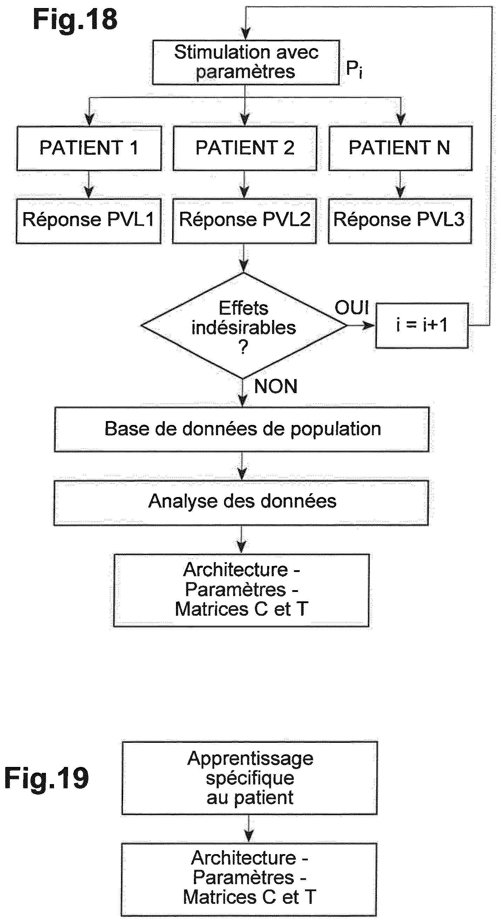

La Figure 18 est un organigramme d'une approche d'apprentissage basée sur une population de patients.

La Figure 19 est un organigramme simplifié d'un mécanisme d'apprentissage spécifique à un patient.

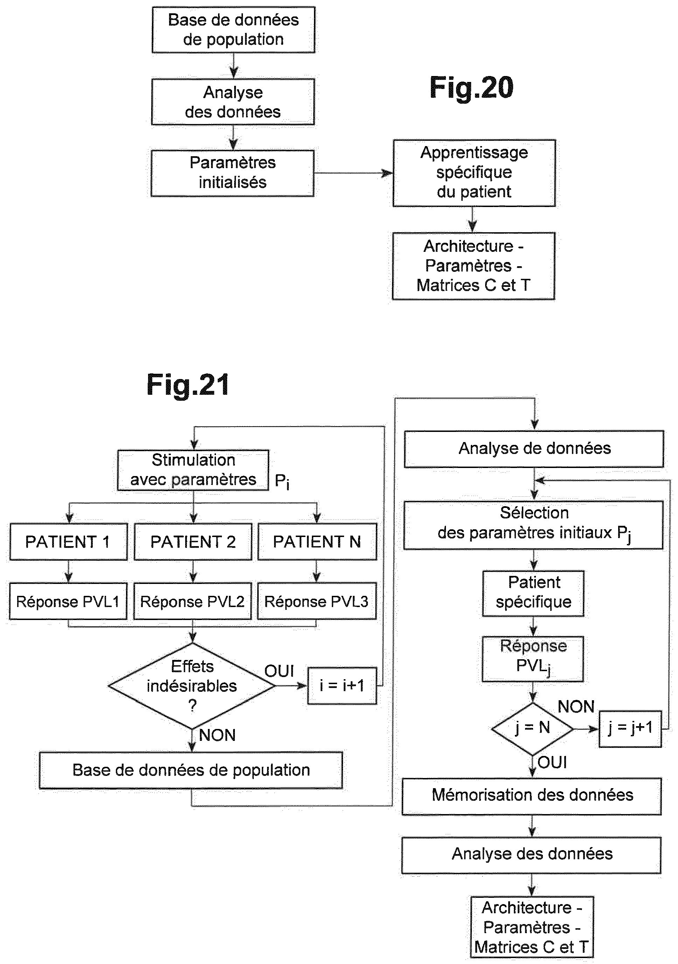

La Figure 20 est un organigramme simplifié illustrant la combinaison d'un apprentissage basé sur une population de patients et d'un apprentissage spécifique à un patient.

La Figure 21 est un organigramme détaillé de cette combinaison.

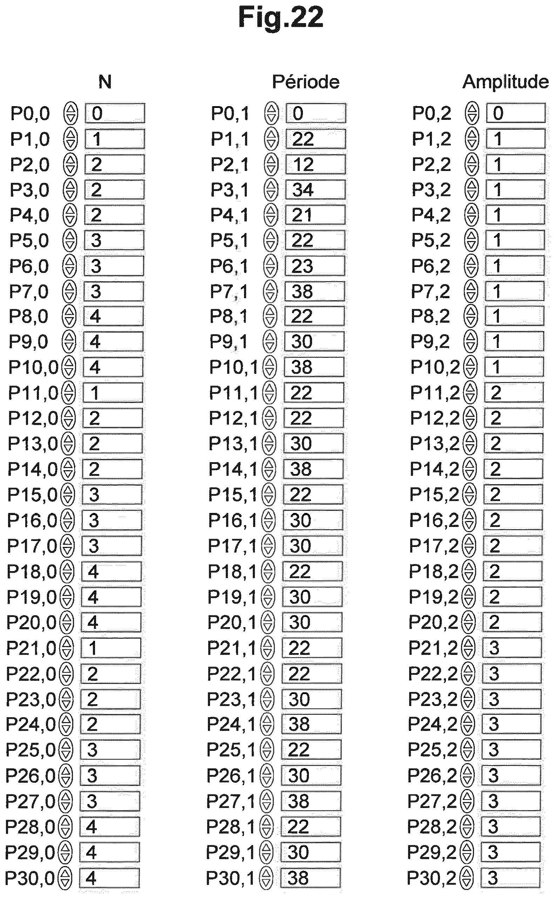

La Figure 22 illustre les paramètres de stimulation pour un ensemble d'états.

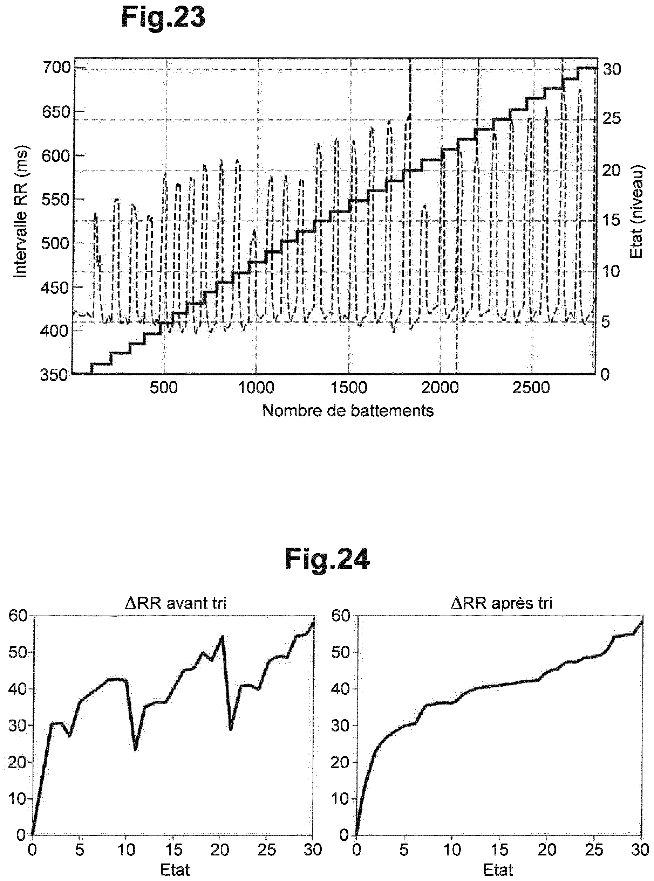

La Figure 23 est un graphe montrant l'évolution dans le temps d'un niveau physiologique et les transitions d'états correspondantes.

La Figure 24 représente des écarts de niveau physiologique, représentant l'effet de la stimulation en utilisant les paramètres associés à chaque état, respectivement avant et après un tri d'états par rapport à l'effet obtenu.

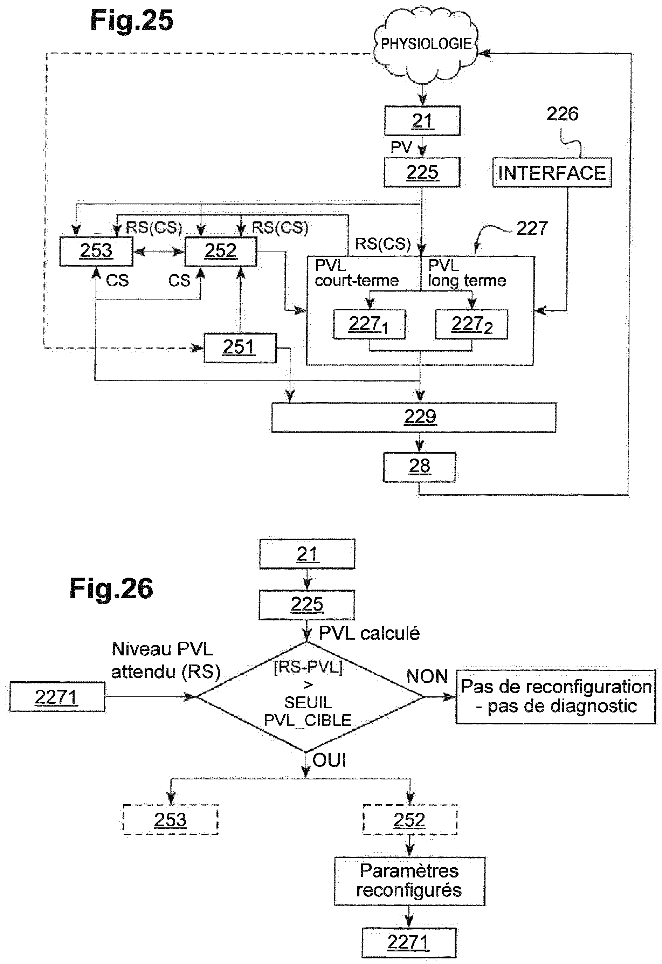

La Figure 25 illustre l'architecture d'un système de commande à transition d'état adaptatif.

La Figure 26 est un organigramme d'une telle commande adaptative.

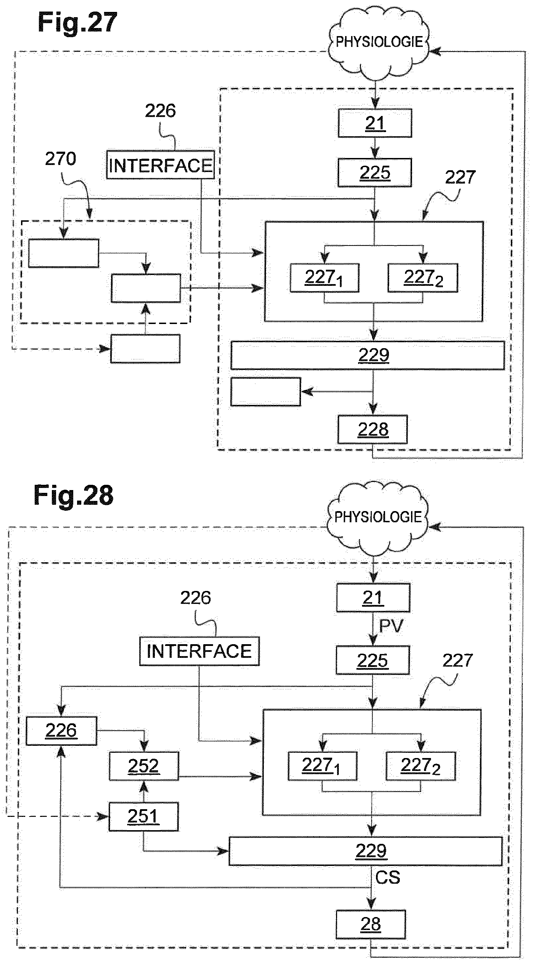

La Figure 27 illustre l'implémentation de l'architecture de la Figure 25 dans le cadre d'une estimation de paramètres déportée.

La Figure 28 illustre l'implémentation de l'architecture de la Figure 25 dans le cadre d'une estimation de paramètres intégrée.

On va maintenant décrire un exemple de réalisation du dispositif de l'invention.

Le procédé de l'invention est mis en oeuvre par des moyens principalement logiciels, au moyen d'algorithmes appropriés exécutés par un microcontrôleur ou un processeur numérique de signal. Pour la clarté de l'exposé, les divers traitements appliqués seront décomposés et schématisés par un certain nombre de blocs fonctionnels distincts présentés sous forme de circuits interconnectés, mais cette représentation n'a toutefois qu'un caractère illustratif, ces circuits comprenant des éléments communs et correspondant en pratique à une pluralité de fonctions globalement exécutées par un même logiciel.

On notera à titre liminaire que dans la mesure du possible, les mêmes signes de référence sont utilisés d'une figure à l'autre pour désigner les mêmes éléments.

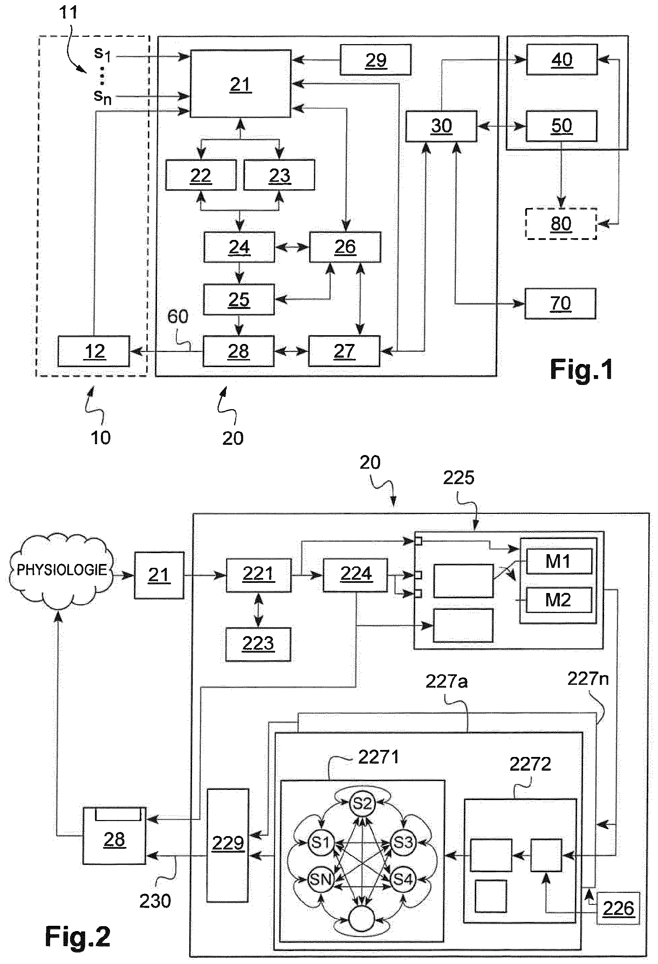

En référence tout d'abord à la Figure 1, on a représenté l'architecture générale d'un système de commande de stimulation thérapeutique auquel s'applique l'invention.

Le dispositif est divisé en trois sous-systèmes principaux :

i) un générateur d'impulsions implantable ou externe 20 comprenant les éléments suivants :

- un module de traitement de données sous forme d'un microcontrôleur ou microprocesseur 27, apte à communiquer avec d'autres modules du dispositif pour recevoir ou envoyer des données et des commandes de contrôle ;
- un module 21 composé d'un ensemble de capteurs et de moyens de traitement de données, apte à enregistrer, à stocker et à traiter les données provenant des capteurs ; ces moyens de traitement comprennent de façon non limitative des amplificateurs, des convertisseurs analogique-numérique, des filtres, des compresseurs de données, des fonctions de transfert ;
- un module de commande en boucle fermée 22 destiné à fournir de façon dynamique et auto-adaptative les paramètres d'une stimulation optimale, en fonction d'un ensemble de valeurs cibles pour le ou chaque niveau de variable physiologique ou physique, ci-après "PVL" (*Physical/Physiological Variable Level*). On décrira ce système dans la suite en référence à la Figure 2 ;
- un module de mémoire 26 apte à stocker les paramètres de fonctionnement des différents modules 21 (acquisition des signaux des capteurs et traitement des données), 22 (calcul PVL), 27, 28 (stimulateur), notamment les valeurs cibles des PVL souhaitées, ci-après *"PVLcible",* pour le fonctionnement de la boucle de contrôle, les données calculées au niveau des différents modules, et des données pour le suivi clinique, sur des échelles de temps différentes ;
- un ou plusieurs capteurs internes 29, par exemple un accéléromètre et/ou un capteur de température ;
- un dispositif de stimulation 28 appliquant une stimulation à une structure physiologique 12, ici le corps humain ; dans les exemples décrits ci-dessous, le dispositif 28 est un module électronique de stimulation du nerf vague ;
- un module de télémétrie 30 apte à communiquer avec un dispositif externe tel qu'un programmateur 50 ou un dispositif de surveillance à distance à domicile 40, ainsi qu'avec un autre dispositif implanté 70, par exemple une sonde autonome, un défibrillateur à cardioversion implantable (ICD) ou un dispositif thérapeutique de resynchronisation cardiaque (CRT).

ii) un ensemble 11 de capteurs implantés mesurant différents signaux physiologiques et physiques $s_1 ... s_n$ de la structure 12, en particulier au moins un paramètre parmi les suivants (liste non limitative) : ventilation-minute, accélération endocardiaque (EA), pression, électrogramme endocavitaire (EGM), électroneurogramme (ENG). Ces signaux sont enregistrés par le module 21 et transformés en variables physiologiques ou physiques (PVL) telles que (liste non limitative) la fréquence cardiaque (HR), la contractilité ventriculaire, la fréquence respiratoire, la densité d'électroneurogramme, la variabilité de la fréquence cardiaque (HRV, *Heart Rate Variability*). Ces données peuvent être stockées dans la mémoire 26 pour permettre une analyse sur différentes échelles de temps ; ainsi des moyennes des paramètres physiologiques ou physiques sur des durées prédéterminées (par exemple 1 minute, 24 heures et une semaine, respectivement) peuvent être calculées ; en particulier, la fréquence cardiaque moyenne est calculée sur les dernières 24 h (variable dite "meanHR24") ;

iii) au moins l'un parmi les dispositifs externes suivants :

- un programmateur 50 fonctionnant sous le contrôle du médecin 80 (ou tout autre personnel médical autorisé) et apte à interroger le générateur d'impulsions 20 et transférer des données dudit générateur d'impulsions vers la mémoire du programmateur, apte également à transférer des données de réglage du programmateur vers le générateur d'impulsions 20, et apte en outre à afficher des rapports sur écran, dans des fichiers, une base de données ou sur imprimante ;
- le système de surveillance à distance à domicile 40 apte à recevoir du générateur d'impulsions 20 certaines données et/ou des alertes à envoyer au médecin via un système de communication utilisant par exemple le réseau téléphonique cellulaire et les stockant dans une base de données.

iv) un effecteur 60, par exemple une sonde de stimulation du nerf vague, reliant le module de stimulation 28 à la structure physiologique à stimuler 12.

Un aspect de l'invention réside dans un système de commande à boucle fermée particulier, représenté sur la Figure 2.

En référence à cette même figure, le système de commande à boucle fermée 22 reçoit en entrée les signaux délivrés par les capteurs physiologiques ou physiques de l'ensemble 21, le cas échéant après un prétraitement, comprenant notamment des signaux ECG acquis à partir d'un dispositif externe, des signaux d'EGM fournis par une sonde cardiaque d'un dispositif implantable, une pression, une accélération globale G, une accélération endocardiaque EA, des signaux d'ENG.

Le système 22 comprend également une unité de traitement de signaux 221, une unité de mémorisation 223, une unité de détection d'événements 224, une unité 225 de calcul du niveau des variables physiques et/ou physiologiques PVL, une interface 226 entre le système de contrôle de transition d'état 227 et d'autres modules de l'appareil, tels que le module de mémoire 26 de la Figure 1 et qui permet, entre autres, d'obtenir la valeur cible de la variable physiologique (*PVLcible*) de la mémoire 26, et un ou plusieurs dispositifs de commande de transition d'état 227 reliés fonctionnellement en 230 à l'unité de stimulation neuronale 28 via un module 229 de fusion de paramètres, tel que décrit dans la suite.

Chaque contrôleur de transition d'état 227 comprend deux éléments interconnectés, à savoir i) un modèle de transitions d'état 2271, contenant tous les états du dispositif de commande de transition d'état et leurs interconnexions, et ii) un calculateur 2272 mettant en oeuvre un ou plusieurs algorithmes de transition d'état.

Le module 229 de fusion de paramètres est apte à déterminer les paramètres finaux de neurostimulation comme on va le décrire dans la suite.

Le dispositif de commande 227 selon l'invention opère dans une configuration à boucle fermée : une variable physiologique et physique courante, ci-après "*PVLcourant*", est mesurée et prétraitée par 225. L'interface 226 contient l'information d'une valeur cible pour cette même variable physiologique (*PVLcible*). Cette valeur *PVLcible* peut-être fixe ou dynamique. Une *PVLcible* fixe peut être définie par défaut dans le dispositif ou encore, modifiée et définie par l'expert et enregistrée dans la mémoire 26. Par exemple, dans le contrôle de l'intervalle RR, l'expert peut définir une *PVLcible* = 500 ms (fixe) dans la mémoire 26. Une *PVLcible* dynamique est définie lorsque sa valeur est fonction d'une variable mesurée. Dans ce cas, la *PVLcible* changera au cours du temps. Par exemple, toujours dans le cas du contrôle de l'intervalle RR, l'expert peut définir une *PVLcible* dynamique comme un pourcentage du RR mesuré (i.e. *PVLcible* = 10%\**PVLcourant*). Le calcul de la fonction sous-jacente à cette définition dynamique de la *PVLcible* est réalisé dans le module 226. Enfin, il est important de souligner que la fonction utilisée pour obtenir une *PVLcible* dynamique peut être tout type de fonction mathématique ou un ensemble de règles logiques.

Une fois PVLcible définie, une erreur est calculée en 227 entre *PVLcourant* et *PVLcible.* Enfin, sur la base de cette erreur, l'algorithme de commande détermine une transition d'états et l'état ainsi obtenu permet de définir les paramètres de neurostimulation.

On va maintenant décrire le dispositif de commande de transition d'état 227.

Son modèle de transition d'état 2271 est composé d'un ensemble de N+1 états notés S0 à SN, où chaque état correspond à un ensemble de paramètres de stimulation et un ensemble de réponses ou effets caractéristiques notées RS.

Un exemple de modèle de transition d'état est le suivant :

$$S0 = [P_{1,0}\ P_{2,0}\ ...\ P_{M,0}\ ;\ RS_{1,0}\ ...\ RS_{Q,0}]$$
$$S1 = [P_{1,1}\ P_{2,1}\ ...\ P_{M,1}\ ;\ RS_{1,1}\ ...\ RS_{Q,1}]$$
$$S2 = [P_{1,2}\ P_{2,2}\ ...\ P_{M,2}\ ;\ RS_{1,2}\ ...\ RS_{Q,2}] \tag{1}$$

$$SN = [P_{1,N}\ P_{2,N}\ ...\ P_{M,N}\ ;\ RS_{1,N}\ ...\ RS_{Q,N}]$$

où Sn est l'état n, avec n = 0 ... N, et est composé de m paramètres de stimulation du nerf vague, notés Pm,n. Les paramètres Pm,n peuvent représenter tout paramètre d'une telle stimulation, par exemple le nombre d'impulsions, l'amplitude des impulsions, la largeur des impulsions, leur fréquence, le retard de stimulation en cas de neurostimulation synchrone d'un événement physiologique (comme l'activité cardiaque), le rapport cyclique des impulsions, le synchronisme des impulsions, etc.

On va considérer ci-dessous un exemple où l'état Sn est composé des paramètres suivants :

- comme paramètres de stimulation, l'amplitude, la fréquence et la largeur des impulsions de stimulation ;
- comme effets caractéristiques, la durée de l'intervalle RR de l'électrocardiogramme et la variation temporelle (dérivée première) de la pression cardiaque.

Les cinq états du modèle sont dans cet exemple les suivants :

```
S0 = [0mA  0Hz   0µs Baseline Baseline]
S1 = [1mA 64Hz 120µs ; 420ms 1.90mmHg/ms]
S2 = [2mA 32Hz 120µs ; 450ms 2.05mmHg/ms]          (2)
S3 = [3mA 25Hz 120µs ; 500ms 2.09mmHg/ms]
S4 = [3mA 12Hz 240µs ; 520ms 2.15mmHg/ms]
```

Dans chaque modèle, l'un des états (état S0) peut être configuré pour arrêter la neurostimulation. Cet état peut être utilisé dans toutes les situations où la stimulation n'est pas nécessaire, y compris dans le cas où des événements indésirables (typiquement toux, douleur, arythmie...) sont détectés.

Chacun des états du modèle de transition d'état est connecté à lui-même, et peut être connecté à tout autre état Sk.

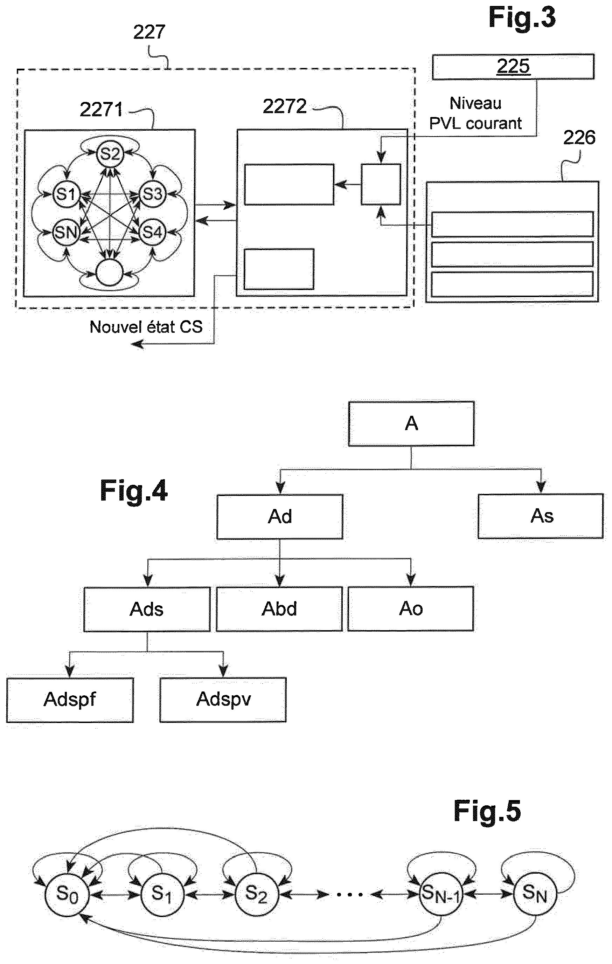

Un exemple particulier, illustré sur la Figure 3, est le cas d'un modèle de transition d'états complètement connecté, à savoir, où chaque état peut transiter vers tout autre état.

Ces connexions entre états, qui gouvernent les transitions entre états, sont ici définies dans une matrice de connexion. La matrice de connexion pour une architecture complètement connectée est illustrée dans le Tableau 1 ci-dessous.

Tableau 1

| État | 0 | 1 | 2 | ... | Nk |
|------|---|---|---|-----|----|
| S0 | 1 | 1 | 1 | 1 | 1 |
| S1 | 1 | 1 | 1 | 1 | 1 |
| S2 | 1 | 1 | 1 | 1 | 1 |
| ⋮ | 1 | 1 | 1 | 1 | 1 |
| SN | 1 | 1 | 1 | 1 | 1 |

Toutes les cellules de la matrice sont ici à la valeur 1, signifiant que tous les états peuvent être connectés les uns aux autres.

La présence d'une valeur 0 dans une cellule de la matrice a pour effet d'interdire une transition directe entre deux états. Ceci peut être utilisé comme moyen de sécurité en interdisant des transitions entre des configurations de stimulation spécifiques. Par exemple, dans le cas de la stimulation du nerf vague (VNS), un changement trop brutal dans le courant injecté peut provoquer des effets indésirables, et un tel changement peut être évité en fixant à une valeur de zéro la ou les cellules correspondantes de la matrice de connexion.

Le calculateur de transition d'état est dédié à la détermination de transitions optimales entre états, de façon à conduire à une optimisation de la configuration des paramètres de neurostimulation par minimisation de l'erreur entre le *PVLcourant* et *PVLcible.*

Les transitions entre états sont définies à des instants spécifiques que l'on dénomme ici "évènements". Ces événements peuvent être réguliers dans le temps (par exemple, à chaque minute), ou répartis de façon synchrone à une activité physiologique (par exemple à chaque battement cardiaque), en fonction d'une situation donnée (par exemple lorsque le patient est endormi, avec une faible variabilité du rythme cardiaque HRV) ou toute combinaison de telles situations. À chaque événement, le calculateur de transition d'état 2272 détermine la transition d'état la mieux appropriée, en appliquant un algorithme de transition d'état basé sur une matrice de transition T comme on va maintenant le voir.

Un exemple de matrice de transition T pour un algorithme de transition séquentiel et déterministe est donné dans le Tableau 2 ci-dessous.

Tableau 2

| Évènememt | | | | | | | | | t+1 |
|---|---|---|---|---|---|---|---|---|---|
| | État | 0 | 1 | 2 | 3 | ... | N-2 | N-1 | N |
| t | S0 | Cond P | Cond I | | | | | | |
| | S1 | Cond P | Cond I | Cond 2 | | | | | |
| | S2 | Cond P | Cond 0 | Cond 1 | Cond 2 | 0 | 0 | 0 | 0 |
| | ... | Cond P | ... | ... | ... | ... | ... | ... | ... |
| | $S_{N-2}$ | Cond P | 0 | 0 | 0 | | Cond 1 | Cond 2 | 0 |
| | $S_{N-1}$ | Cond P | 0 | 0 | 0 | 0 | Cond 0 | Cond 1 | Cond 2 |
| | $S_N$ | Cond P | 0 | 0 | 0 | 0 | 0 | Cond 0 | Cond F |

On observe dans ce tableau l'état S0 qui correspond à l'arrêt immédiat de la neurostimulation dans certaines conditions, par exemple lors de la survenance d'événements indésirables (typiquement la toux, une douleur, une arythmie cardiaque...). C'est une condition de priorité (notée Cond P) qui conduit à cet état, où les paramètres de neurostimulation sont amenés à 0. La condition P est prioritaire sur toutes les autres.

Outre cette condition prioritaire, différents types de conditions peuvent être appliquées. Un exemple simplifié de ces conditions est le suivant :

```
Condition I: PVLi,j courant >= PVLi,j Cible

Condition 0: PVLi,j courant > PVLi,j Cible

Condition 1: PVLi,j courant = PVLi,j Cible        (3)

Condition 2: PVLi,j courant < PVLi,j Cible

Condition F: PVLi,j courant <= PVLi,j Cible

Condition P: condition de priorité
```

où i et j représentent respectivement l'état actuel et l'état futur.

Ces conditions sont données à titre d'exemple illustratif et simplifié. L'homme du métier comprendra que les conditions peuvent être complexes, en mettant en jeu des combinaisons de valeurs cibles et des opérateurs logiques ou fonctionnels entre variables mesurées.

Il est à noter que la matrice de transition pour l'algorithme déterministe est composée d'un ensemble de conditions qui peuvent être mises en oeuvre en tant que règles. L'invention permet aussi de traiter les transitions de façon probabiliste. Dans ce cas, un exemple de matrice de transition T de type stochastique peut être le suivant :

Tableau 3

| Évènement | | | | | | | | t+1 |
|---|---|---|---|---|---|---|---|---|
| | État | S1 | S2 | S3 | ... | $S_{N-2}$ | $S_{N-1}$ | $S_N$ |
| t | S1 | $a_{1,1}$ | $a_{1,2}$ | $a_{1,3}$ | $a_{1,...}$ | $a_{1,N-2}$ | $a_{1,N-1}$ | $a_{1,N}$ |
| | S2 | $a_{2,1}$ | $a_{2,2}$ | $a_{2,3}$ | $a_{2,...}$ | $a_{2,N-2}$ | $a_{2,N-1}$ | $a_{2,N}$ |
| | S3 | $S_{3,1}$ | $a_{3,2}$ | $a_{3,3}$ | $a_{3,...}$ | $a_{3,N-2}$ | $a_{3,N-1}$ | $a_{3,N}$ |
| | - | - | - | - | - | - | - | - |
| | $S_{N-2}$ | $a_{N-2,1}$ | $a_{N-1,2}$ | $a_{N-2,3}$ | $a_{N-2,...}$ | $a_{N-2,N-2}$ | $a_{N-2,N-1}$ | $a_{N-2,N}$ |
| | $S_{N-1}$ | $a_{N-1,1}$ | $a_{N-1,2}$ | $a_{N-1,3}$ | $a_{N-1,...}$ | $a_{N-1,N-2}$ | $a_{N-1,N-1}$ | $a_{N-1,N}$ |
| | $S_N$ | $a_{N,1}$ | $a_{N,2}$ | $a_{N,3}$ | $a_{N,...}$ | $a_{N,...,N-2}$ | $a_{N,N-1}$ | $a_{N,N}$ |

où :

$$\sum_{j=1}^{N} a_{1,j} = 1, \sum_{j=1}^{N} a_{2,j} = 1, \dots, \sum_{j=1}^{N} a_{N,j} = 1 \qquad\qquad (4)$$

et où chaque $a_{i,j}$ représente la probabilité de transition de l'état i à l'état j.

La Figure 4 montre la hiérarchie entre un certain nombre d'exemples d'algorithmes *A* de transition qui vont être décrits dans la suite, avec un algorithme stochastique *As,* un algorithme déterministe *Ad,* et dans cette dernière catégorie un algorithme de transition séquentiel *Ads,* qui peut être de type à pas fixe *Adspf* ou à pas variable *Adspv,* un algorithme basé sur la dichotomie *Abd*, et un algorithme optimal *Ao.*

On donne dans le Tableau 4 ci-dessous un exemple de matrice de connexion C pour un algorithme de transition d'état de type séquentiel avec un état d'arrêt de stimulation :

Tableau 4

| État | 0 | 1 | 2 | 3 | 4 | ... | N-1 | N |
|------|---|---|---|---|---|-----|-----|---|
| S0 | 1 | 1 | 0 | 0 | 0 | 0 | 0 | 0 |
| S1 | 1 | 1 | 1 | 0 | 0 | 0 | 0 | 0 |
| S2 | 1 | 1 | 1 | 1 | 0 | 0 | 0 | 0 |
| S3 | 1 | 0 | 1 | 1 | 1 | 0 | 0 | 0 |
| S4 | 1 | 0 | 0 | 1 | 1 | 1 | 0 | 0 |
| ⋮ | 1 | - | - | - | - | - | - | - |
| SN-1 | 1 | 0 | 0 | 0 | 0 | 1 | 1 | 1 |
| SN | 1 | 0 | 0 | 0 | 0 | 0 | 1 | 1 |

On comprend à partir de ce tableau que chaque état du modèle de transition d'état est connecté à S0, à lui-même et à ses états adjacents, comme illustré sur la Figure 5.

L'état S0 correspond à un arrêt immédiat de la neurostimulation dans le cas d'événements indésirables, comme décrit précédemment.

Dans l'un des modes de réalisation, les états du modèle de transition d'état sont catégorisés en fonction de l'effet qu'ils provoquent sur la ou les variables physiologiques à réguler. Les états S0 à S4 donnés plus haut à titre d'exemple (2) sont un exemple d'états catégorisés vis-à-vis de la régulation de l'intervalle RR et de la variation temporelle (dérivée première) de la pression cardiaque.

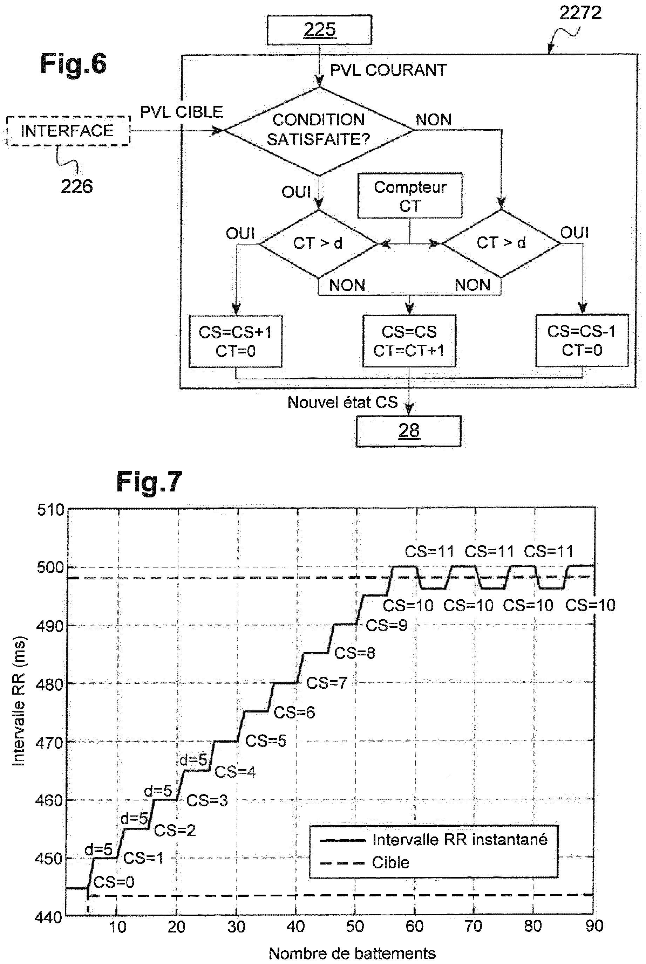

Un exemple d'algorithme de transition d'état déterministe et séquentiel avec pas fixe *Adspf* est illustré sur la Figure 6. Dans ce cas, le pas de 1 correspond à la transition d'un état i à un état j immédiatement adjacent.

Le compteur (CT) permet une remise à jour de l'état tous les d évènements, par exemple tous les 5 cycles cardiaques.

Un exemple de la façon dont cet algorithme doit opérer pour réguler l'intervalle RR instantané d'un patient est illustré sur la Figure 7. Dans cet exemple, la valeur cible de l'intervalle RR est fixée à 498 ms et la valeur d est fixée à 5 cycles cardiaques.

Cette même Figure montre que l'état courant (CS) augmente tous les 5 battements jusqu'à ce que la valeur observée de l'intervalle RR soit supérieure à la valeur cible ($CS_{11}$). Ensuite, l'état courant CS oscille entre les valeurs $CS_{11}$ et $CS_{10}$. La période d'oscillation est de 5 battements.

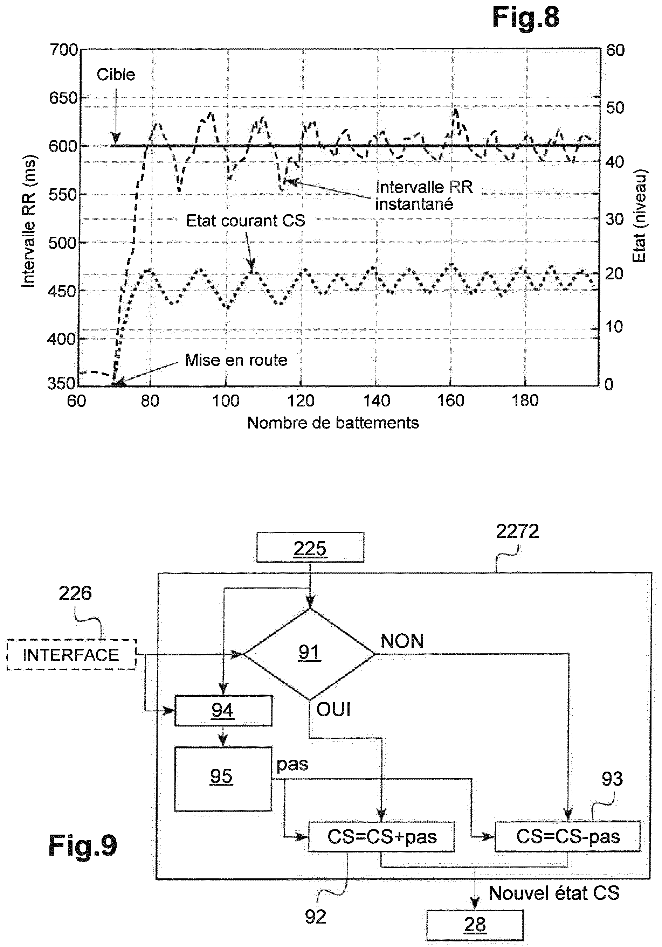

Dans un enregistrement préclinique réel, le résultat de l'application de l'algorithme est montré sur la Figure 8. La valeur cible de l'intervalle RR est fixée à 600 ms et d = 4 cycles cardiaques. On observe que la valeur cible de l'intervalle RR est atteinte.

Les avantages de l'algorithme *Adspf* sont que la valeur cible peut être atteinte avec une bonne précision et que l'algorithme de transition est simple.

Une version plus sophistiquée de l'algorithme déterministe séquentiel Ads est illustrée sur la Figure 9.

Avec cet algorithme, si la condition du dispositif de commande est satisfaite (par exemple si la valeur *PVLcourant* est inférieure à la valeur de *PVLcible,* boite 91), alors l'algorithme exécute (boite 92) CS=CS+pas. Dans le cas contraire, l'algorithme exécute (boite 93) CS=CS-pas. Dans ce cas, la valeur de pas, qui est mise à jour à chaque événement, dépend de l'amplitude de l'erreur, soit :

$$e = PVLcible - PVLcourant$$
$$pas = f(e)$$

calculée à la boite 94.

La boite 95 détermine la valeur du pas. En général, plus la valeur de e est grande, alors plus la valeur de pas est importante. Inversement, plus la valeur de e est petite plus la valeur de pas est petite. Dans certains modes de réalisation, la valeur de pas peut résulter de l'application d'une fonction non linéaire sur la valeur de l'erreur e (par exemple de type sigmoïde) ; dans d'autres modes de réalisation, la valeur de pas peut résulter de l'application d'une fonction linéaire sur la valeur de l'erreur e bornée aux limites mini et maxi du pas.

Des avantages de cette implémentation sont que l'objectif est en général atteint avec précision, et qu'il est plus rapide que les algorithmes décrits plus haut.

On va maintenant décrite un mode de réalisation avec algorithme de transition déterministe basé sur la dichotomie *Abd.* Dans ce cas, le calculateur de transition d'état 2272 opère selon un principe dichotomique, comme illustré sur la Figure 10.

Sur cette figure, deux registres CSmax et CSmin sont initialisés avec CSmax = N et CSmin = 0. Et l'état courant CS est initialisé à la valeur CS = (CSmax-CSmin)/2 (boite 101). Lorsque le système de commande est mis en route, le dispositif de stimulation délivre la stimulation. Ensuite, la nouvelle valeur de *PVLcourant,* qui a déjà été affectée par la stimulation, est calculée à partir des signaux relevés. Si la condition du dispositif de commande est satisfaite (par exemple si la valeur mesurée (*PVLcourant*) est inférieure à la valeur de *PVLcible,* boite 102), alors la variable CSmin est mise à jour, à savoir CSmin = CS, et l'état courant CS est calculé avec CS = CSmin+(CSmax-CSmin)/2 (boite 103). Sinon, la variable CSmax est mise à jour, à savoir CSmax = CS, et l'état courant est calculé avec CS = CSmin+(CSmax-CSmin)/2 (boite 104).

Dans l'algorithme illustré sur la Figure 10, la condition suivante (non illustrée) doit être prise en compte : si CS = CSmin+(CSmax-CSmin)/2 n'est pas un nombre entier, alors CS doit être arrondi à la valeur entière la plus proche.

Les avantages d'un tel algorithme à base dichotomique sont l'atteinte de l'objectif avec précision, et, en général, avec une plus grande rapidité que les algorithmes décrits plus haut.

On va maintenant décrire en référence à la Figure 10bis un mode de réalisation d'un algorithme de transition d'état déterministe optimal Ao. Dans ce mode de réalisation, le calculateur de transition d'état 2272 utilise une valeur de réponse attendue RS précédemment mémorisée dans le modèle de transition d'état 2271 (on rappellera ici que dans les différentes formes de réalisation, chaque état du modèle de transition d'état 601 contient la ou les réponses attendues pour les paramètres de l'état, voir liste (1) plus haut).

Dans cette approche, lorsqu'une valeur *PVLcible* est définie, le calculateur de transition d'état 2272 recherche la réponse attendue RS minimale pour laquelle la condition suivante du dispositif de commande

error(PVLcible, PVLcourant)>thresholdCL

(testée à la boite 105) est remplie et où thresholdCL est défini par un expert.

Dans l'affirmative, cela signifie que l'état courant CS est l'état contenant cette valeur de RS.

Par exemple, supposons que l'on utilise ici le modèle de transition d'état à 4 états actifs figurant dans la liste (2) plus haut, à savoir :

$$S1 = [1mA \ 64Hz \ 120\mu s \ ; \ 420ms \ 1.90mmHg/ms]$$
$$S2 = [2mA \ 32Hz \ 120\mu s \ ; \ 450ms \ 2.05mmHg/ms]$$
$$S3 = [3mA \ 25Hz \ 120\mu s \ ; \ 500ms \ 2.09mmHg/ms]$$
$$S4 = [3mA \ 12Hz \ 240\mu s \ ; \ 520ms \ 2.15mmHg/ms]$$

Supposons maintenant que l'on s'intéresse à la régulation de l'intervalle RR sur une valeur cible de 440 ms. L'algorithme de transition va alors choisir comme état courant S2, car cet état S2 est la valeur la plus basse qui remplisse la condition :

RS > PVLcible.

Comme décrit précédemment un état S0 peut être utilisé pour des conditions de sécurité.

Un avantage de cet algorithme est que l'objectif est généralement atteint avec précision, et de façon plus rapide qu'avec les algorithmes précédemment exposés.

On notera ici que la matrice de connexion C telle que montrée dans le tableau 1 peut être utilisée avec l'algorithme de transition déterministe et séquentiel à pas variable *Adspv,* avec l'algorithme de transition déterministe à base dichotomique *Abd* et avec l'algorithme de transition déterministe optimal *Ao.*

On va maintenant décrire un exemple de réalisation utilisant un algorithme de transition stochastique *As*.

Alors que l'approche déterministe optimale décrite ci-dessus est basée sur l'hypothèse qu'une configuration de stimulation donnée Pi générera toujours l'exacte réponse attendue RSi, ceci n'est en réalité jamais vrai en physiologie du fait de la complexité de ces systèmes.

Une approche stochastique selon ce mode de réalisation vise à traiter une partie de l'incertitude liée à cette complexité en appliquant une probabilité de transition à un état donné.

Dans tous les modes de réalisation du calculateur de transition d'état 2272 opérant selon des lois probabilistes, des graphes probabilistes peuvent être utilisés pour calculer la transition d'état à sélectionner à partir du modèle de transition d'état.

Un graphe probabiliste est un graphe orienté et pondéré pour lequel :

- il existe au moins un arc d'un état vers un autre ou vers lui-même.
- la somme des poids des arcs partant du même état est égale à 1 (équations 4).

On notera que les poids sont alors des probabilités, à savoir des nombres réels compris entre 0 et 1 et qu'un graphe probabiliste montre les états possibles du système et les probabilités de transition d'un état à un autre (poids des arcs).

La Figure 11a est un exemple de graphe probabiliste d'ordre deux (à deux états). La Figure 11b est un exemple d'un graphe probabiliste d'ordre trois. La Figure 11c montre un exemple d'un graphe non probabiliste, puisque la somme des poids des arcs partant de l'état S1 est égale à 0,9 et non à 1.

Dans un mode de réalisation, une matrice de Markov est utilisée pour déterminer les transitions optimales permettant d'atteindre le niveau cible de la variable physiologique, tel que décrit dans le tableau 3, où $a_{i,j}$ représente la probabilité d'une transition d'un état vers un autre dans le modèle de transition d'état.

Comme décrit précédemment, un état S0 peut être prévu pour des conditions de mise en sécurité.

Dans un mode de réalisation, les probabilités $a_{i,j}$ sont calculées pendant une phase d'apprentissage, via des algorithmes d'un modèle de Markov caché (HMM, *Hidden Markov Model*). Par exemple, après avoir fixé le nombre d'états, l'algorithme de Baum-Welch (*forward-backward*) permet d'estimer l'ensemble de paramètres du modèle de transition d'états (ici un HMM) de façon itérative, à partir d'une base de données d'apprentissage. D'autres algorithmes, comme le *Iterated Conditional Estimator* peuvent être également utilisés. Une fois ces paramètres identifiés, à partir d'une séquence d'observations, l'algorithme de Viterbi calcule la séquence d'états qui sera le plus probablement correspondante, tandis que l'algorithme *forward* calcule la probabilité d'une séquence particulière d'observations.

Un exemple de diagramme de l'effet de cet algorithme de transition est illustré sur la Figure 12. La valeur cible de l'intervalle RR est fixée à 500ms. Un modèle de transition d'état (HMM) à N états, avec N = 10, est utilisé. On observe que l'algorithme de transition conduit à des états CS = S3, puis CS = S2, puis amène directement à l'état CS = S5, qui ici, en fonction des conditions, est à même de mieux conduire le niveau de la variable physiologique vers sa valeur cible.

On comprend que dans cet exemple, il n'y a pas de règles particulières comme dans les algorithmes déterministes. Dans cet exemple, les transitions sont fonction des probabilités associées à la matrice de transition T. On observe en outre que les états peuvent varier d'une manière probabiliste, même si la valeur physiologique cible reste constante. Ce comportement probabiliste peut être particulièrement important dans des applications physiologiques ou cliniques du fait que les processus sous-jacents sont complexes et présentent donc une variabilité naturelle qui peut être représentée par un processus aléatoire.

On va maintenant décrire une forme de réalisation avec commande à boucle fermée à résolution temporelle variable.

On sait qu'un dispositif de neurostimulation adaptatif doit être capable d'adapter le traitement à la fois à des variables susceptibles de changer rapidement (échelle de temps de la seconde ou de la minute) et à des variables susceptibles de changer lentement (échelle de temps du jour, du mois, de l'année). Dans cette forme de réalisation, on utilise par exemple deux échelles de temps :

- les variables physiologiques à changement rapide sont souvent des variables régulées à court terme par le système nerveux autonome, telles que la fréquence cardiaque, la pression artérielle, la contractilité. Elles sont liées à l'évolution de l'activité du patient ou à une aggravation aigüe de l'état du patient (comme l'infarctus du myocarde) ; d'autres variables de court terme sont liées à la survenue d'événements indésirables (toux, contraction des muscles du cou, etc.). Ces variables nécessitent une réaction immédiate, telle qu'une interruption de la neurostimulation ;
- les variables physiologiques à changement lent ou dynamique de long terme sont liées :

  - à des mécanismes physiologiques lents, correspondant à une amélioration ou à une dégradation de l'état du patient, telle que la modification de la performance hémodynamique cardiaque, résultant en des changements de gain des voies autonomes; ils peuvent être évalués notamment par le rythme cardiaque moyen, l'évolution de la variabilité du rythme cardiaque HRV, ou un changement de l'équilibre sympathovagal (SVB), mais aussi par l'activité moyenne du patient entre autres par la mesure par un accéléromètre ;

- à des phénomènes électromécaniques, comme l'évolution du couplage entre électrode et nerf du fait d'une fibrose évolutive, généralement après la mise en place de l'électrode de stimulation.

La thérapie par neurostimulation est alors adaptée pour suivre ces variations à résolutions temporelles différentes.

La multirésolution temporelle est assurée dans cette forme de réalisation, comme représenté sur la Figure 13, en prévoyant plusieurs dispositifs de commande de transition d'état, respectivement $227_1$, $227_2$... $227_K$, chaque dispositif opérant avec une résolution temporelle différente et avec des paramètres du modèle de transition d'état et des algorithmes différents, tels que définis précédemment.

Pour illustrer cette particularité, on va décrire ici une version du système de commande intégrant K dispositifs de commande de transition d'état, avec ici K = 2.

Ainsi le système de contrôle est composé de deux dispositifs de commande de transition d'état $227_1$ et $227_2$, l'un assurant une régulation d'une variable physiologique sur le court terme (notée PV1), et l'autre assurant une régulation d'une variable physiologique sur le long terme (notée PV2), et les changements dans cette variable PV2 affectant l'effet d'une stimulation du nerf vague (VNS) sur la variable PV1.

Dans de telles circonstances, l'effet d'une stimulation VNS donnée peut changer dans le temps, comme illustré sur la Figure 14. Sur cette Figure, M0 représente l'effet de la stimulation VNS sur la variable PV1 pendant l'implantation, M3 représente l'effet de la VNS sur la variable PV1 trois mois après l'implantation, et M6 représente l'effet de la stimulation VNS sur la variable PV1 six mois après l'implantation. Les trois courbes illustrées sur la Figure 14 ont été obtenues avec les mêmes cinq configurations de paramètres de stimulation VNS successives.

Dans cet exemple, un premier dispositif de commande est utilisé pour réguler la variable PV1, tandis que le second dispositif de commande estime l'effet de la stimulation sur la variable PV1 et compense les variations de cet effet sur l'échelle de temps M0, M3, M6.

La Figure 15 montre un exemple de la façon dont le système de commande peut être mis en oeuvre, les dispositifs de commande $227_1$, et $227_2$ délivrant des consignes O1 et O2 qui sont fusionnées au niveau du module de fusion 229 pour déterminer l'état courant vers lequel effectuer une transition. Dans cet exemple, cette fusion consiste en une multiplication.

La Figure 16a montre en partie supérieure la réponse de la variable PV1 lorsqu'on utilise un dispositif de commande unique. La Figure 16b montre en partie supérieure la réponse de la variable PV1 lorsqu'on utilise deux dispositifs de commande. Les différentes courbes correspondent à différents moments (en mois, jours, années ou autres) auxquels une valeur *PVLcible* de la variable physiologique PV1 est établie.

On observe en bas de la Figure 16a que lorsqu'un seul contrôleur est utilisé, la sortie du dispositif de commande $227_1$ conduit à des paramètres de stimulation différents pour atteindre la cible. Ceci s'explique par l'impact des variations de la variable PV2 sur l'effet de la stimulation VNS sur la variable PV1.

Au contraire, et comme illustré en bas de la Figure 16b, lorsque deux dispositifs de commande $227_1$, $227_2$ sont utilisés, la sortie du dispositif de commande $227_1$ conduit à des paramètres de stimulation de valeurs semblables pour atteindre la cible, du fait de la compensation assurée par le deuxième dispositif de commande $227_2$. L'approche à deux dispositifs de commande permet d'obtenir une meilleure précision et des temps de convergence plus faibles, tout en utilisant un modèle de transition d'états plus simple (nombre d'états plus faible).

Un cas particulier de contrôle à plusieurs dispositifs de commande est le cas où un ou plusieurs modèles de transition d'états probabilistes sont utilisés pour améliorer la précision autour d'une zone de valeurs de la *PVLcible* et un ou plusieurs modèles de transition d'états déterministes sont utilisés pour améliorer la rapidité de convergence (temps nécessaire pour atteindre la cible). Dans ce cas, chaque dispositif de contrôle aura un modèle probabiliste ou déterministe différent et le module de fusion 229 définira les paramètres définitifs de stimulation.

On va maintenant décrire des algorithmes avec phase d'apprentissage.

Il est bien connu dans le domaine de la stimulation du nerf vague que différents patients répondent différemment à la même configuration de stimulation VNS. Par conséquent, une thérapie VNS (à savoir les différents paramètres de stimulation et donc, différents états possibles du modèle de transition d'état) est ici adaptée durant une phase d'apprentissage spécifique au patient. Cette phase d'apprentissage peut prendre beaucoup de temps, et dans certains cas, il n'est pas possible de la mettre en oeuvre. Dans de tels cas, on utilise une configuration de stimulation VNS généralisée (sous forme d'une table d'états générale) dérivée de l'analyse d'une base de données d'une population similaire à celle du patient traité. Dans un mode de réalisation plus sophistiqué, cette table d'états générale peut être utilisée comme table d'états initiale, et une phase d'apprentissage spécifique au patient peut être réalisée de façon à améliorer la précision du système de commande.

Cette forme de réalisation se base avantageusement sur une approche à boucle fermée intégrant des propriétés spécifiques au patient.

Afin de mettre en oeuvre le ou les dispositifs de commande de transition d'état, il est tout d'abord nécessaire d'identifier les paramètres principaux du modèle de transition d'état associé à partir de données de population, ou d'une manière spécifique au patient.

En référence à la Figure 17, l'architecture du dispositif de commande et les paramètres des modèles de transition d'état, principalement les matrices C et T, sont définis (étape 171) en mettant en oeuvre une phase d'apprentissage 172 qui peut être basée sur une population (étape 173) ou spécifique au patient (étape 174).

La structure de chaque dispositif de commande de transition d'état est caractérisée par la définition du nombre d'états du modèle et par les connexions inter-états souhaitées (telles que définies dans la matrice C). Ces définitions sont fixées à partir d'une analyse des besoins. Une fois cette structure définie, les paramètres de l'algorithme de transition d'état (principalement le jeu de paramètres de stimulation $P_{Mk,Nk,k}$ et les valeurs atteintes $RS_{Qk,Nk,k}$ pour chaque état, ainsi que la matrice de transitions T, doivent être déterminés.

D'une façon générale, la phase d'apprentissage consiste à stimuler le patient (ou plusieurs patients) avec un ensemble de paramètres de stimulation et à analyser les valeurs atteintes de variables physiologiques avec ces jeux de paramètres. A partir de ces observations, une analyse est réalisée afin de définir la structure et les paramètres de chaque modèle de transition d'état et en particulier les matrices C et T. De plus, cette phase d'apprentissage permet de détecter des paramètres qui provoquent des effets secondaires, de manière à les exclure du modèle de transition d'état.

Une phase d'apprentissage basée sur la population est illustrée sur la Figure 18. Cette approche est basée sur l'analyse d'une base de données d'une population, typiquement caractéristique de la pathologie visée, comprenant des enregistrements des variables physiologiques d'intérêt, pour différents patients, et avec différents paramètres de stimulation pour chaque patient. Les paramètres $P_{Mk,Nk,k}$ et les valeurs atteintes $RS_{Qk,Nk,k}$ pour chaque état (constituant l'ensemble $Sn_k$,k), ainsi que la matrice T, sont obtenus par des analyses de sensibilité sur les données disponibles. La Figure 18 illustre l'organigramme complet de cette phase d'apprentissage basée sur la population.

Comme indiqué plus haut, les paramètres qui sont susceptibles de provoquer des effets indésirables ne sont pas utilisés dans le modèle de transition d'état (cf. test d'effets indésirables de la Figure 18).

Le schéma-bloc d'une phase d'apprentissage spécifique au patient est illustré sur la Figure 19. Dans le but d'évaluer l'effet de chaque jeu de paramètres de stimulation (Snk) sur les variables physiologiques PVLs d'un patient particulier, cette procédure d'apprentissage spécifique au patient peut être appliquée dans un cadre peropératoire ou post-opératoire, typiquement lors des visites de contrôle. Dans cette procédure, le patient est stimulé en utilisant un ensemble de paramètres de stimulation, avec un balayage des valeurs de paramètres, et l'effet sur les variables PVLs (ou sur une variable principale mise en jeu) est mesuré. Les paramètres. $P_{Mk,Nk,k}$ et les valeurs atteintes $RS_{Qk,Nk,k}$ pour chaque état (constituant l'ensemble $Sn_k$,k), ainsi que la matrice T, sont obtenus en analysant ces données.

Ici encore, les paramètres qui sont susceptibles de provoquer des effets indésirables ne sont pas utilisés dans le modèle de transition d'état.

Il est possible de combiner les approches mentionnées ci-dessus (i) en commençant par une définition de paramètres basée sur la population et (ii) en affinant ces paramètres lors d'une session d'analyse per- et/ou post-opératoire, comme illustré sur la Figure 20.

Un programme complet de cette procédure d'apprentissage combiné est illustré sur la Figure 21.

On notera que ces approches d'apprentissage peuvent être mises en oeuvre pour chaque dispositif de commande de transition d'état K, lors d'un agencement avec plusieurs dispositifs de commande.

Une fois cette phase d'apprentissage achevée, tous les paramètres sont stockés dans la mémoire 223 du dispositif de stimulation et l'algorithme de commande, de type déterministe, stochastique ou combiné comme décrit plus haut, peut commencer à opérer sur la base de ces paramètres.

On va maintenant décrire un exemple de phase d'apprentissage appliquée sur un modèle animal :

1) initialisation (Figure 22): le modèle de transition d'état est initialisé avec des paramètres de stimulation provenant de l'analyse d'une base de données de population ; sur cette Figure 22, la première colonne indique le nombre N d'impulsions de stimulation du nerf vague VNS, la deuxième colonne indique la période des impulsions en ms, et la troisième colonne indique l'amplitude des impulsions (sous la forme du courant d'impulsions exprimé en mA) ;

2) apprentissage (Figure 23): le nerf vague est stimulé en utilisant chaque jeu de paramètres VNS du modèle de transition d'état initialisé ; dans le même temps, la valeur attendue de PVL ($RS_{Qk,Nk,k}$) correspondant à chaque jeu de paramètres VNS ($P_{Mk,Nk,k}$) est associée à l'état correspondant ; dans cet exemple particulier, chaque configuration de stimulation VNS est appliquée pendant 15 secondes et la valeur moyenne de l'intervalle RR ($RR_{stim}$) est calculée à partir des cinq derniers battements ; une période de repos (absence de stimulation pendant ici 45 secondes), permet à l'intervalle RR de revenir à la ligne de base ($RR_{rest}$); les variations de l'intervalle RR, notées $\Delta RR$, sont calculées à partir de l'équation suivante :

$$\Delta RR = 100 \times \frac{RR_{rest} - RR_{stim}}{RR_{rest}}$$

où:

$$RR_{rest} = \frac{1}{4}\sum_{i=1}^{4} RR_i$$

$$RR_{stim} = \frac{1}{N-5+1}\sum_{i=5}^{N} RR_i$$

3) tri des paramètres : après l'apprentissage, les paramètres de la stimulation VNS sont triés dans l'ordre croissant, en fonction des valeurs de ΔRR (voir Figure 24 : partir de gauche avant tri, partie de droite après tri). Il est important de souligner ici qu'une étape de fusion d'états peut être réalisé à ce stade. Par exemple, si deux états consécutifs (après tri) provoquent des effets comparables (états 15 et 16, figure 24b, on peut retenir l'un seul de ces états et réduire ainsi le nombre d'états global du modèle de transition.

On notera que les phases d'apprentissage et de tri sont nécessaires afin d'assurer le bon fonctionnement de l'algorithme de transition d'état déterministe et séquentiel, étant donné qu'un tel algorithme est basé sur le principe selon lequel l'effet mesuré de chaque état courant (CS) de stimulation est d'autant plus grand que le niveau de stimulation de l'état courant en question est important.

On va maintenant décrire un système de stimulation adaptatif, réalisant une mise à jour des paramètres du système de commande au cours du temps.

On comprend que les paramètres définis pour chaque dispositif de commande au début d'une thérapie peuvent s'avérer non optimaux à un moment ultérieur, du fait que l'état physiologique et physique du patient est en constante évolution en raison de différents facteurs prévisibles tels que les rythmes circadien et saisonnier, ou imprévisibles comme les modifications de l'environnement (température, socialité...), les maladies chroniques ou aiguës (grippe saisonnière, cancer, insuffisance cardiaque...), le comportement (alimentation, activité, consommation d'alcool, tabagisme...), les événements indésirables.

Afin de fournir la thérapie optimale, l'évolution de la pathologie du patient peut nécessiter de mettre à jour régulièrement les principaux paramètres du modèle de transition d'état. C'est ce que permet l'approche d'estimation adaptative des paramètres proposée ici, qui peut en outre être appliquée à différentes résolutions temporelles, par exemple tous les jours, une fois par semaine, une fois par mois, etc. Cette mise à jour des paramètres du dispositif de commande peut aussi être mise en oeuvre en fonction de l'activité du patient (exercice, sommeil...). Ainsi, en implémentant cette approche adaptative, il est possible d'éviter ou de réduire les visites médicales et de délivrer de façon permanente la thérapie optimale.

En effet, chaque fois que le patient est stimulé avec un ensemble de paramètres donné, le dispositif implantable va obtenir l'effet réel sur le patient en utilisant le calculateur 225 de niveau de la variable physiologique. Cet effet est censé être proche de la valeur attendue RS1.

Toutefois, en raison de la variabilité intra-patient, de la progression d'une pathologie et de l'effet de la thérapie, ces valeurs peuvent devenir significativement différentes. Et lorsque la différence entre ces valeurs devient supérieure à un seuil noté PVL THRES, il peut être décidé (i) d'enregistrer cet événement comme élément d'analyse du système et (ii) d'activer un algorithme d'adaptation qui va mettre à jour les paramètres du dispositif de commande.

La Figure 25 illustre en détail l'architecture de cette configuration adaptative. Sur cette Figure et comme décrit précédemment, les mesures de valeurs PV délivrées par l'unité 21 sont utilisées pour calculer différents niveaux de valeurs physiologiques PVL à l'aide du calculateur de niveaux PVL 225 (par exemple et de façon non limitative l'intervalle RR, les pics maximaux de variation de la pression artérielle notés $(dP/dt)_{max}$, l'activité physique). Ces variables sont introduites dans le système de commande proposé, composé d'un ou de plusieurs dispositifs de commande de transition d'état, ici deux dispositifs $227_1$ et $227_2$.

Chaque dispositif de commande de transition d'état génère un jeu de paramètres possibles fonction du niveau PVL reçu du calculateur. Un module de capteurs d'activité 251 détecte l'activité du patient pendant la journée, par exemple en la qualifiant sur différents niveaux "repos", "marche", "sommeil", "alimentation". Ces informations d'activité sont utilisées par un module de reconfiguration des paramètres 252 mettant en oeuvre un algorithme de reconfiguration dédié et par le module 229 de gestion de la fusion de paramètres, afin d'adapter le modèle de transition d'état et le traitement à l'activité du patient.

Le module de gestion de la fusion des paramètres sélectionne le jeu optimal de paramètres de stimulation VNS à partir d'une série de jeux possibles de paramètres afin d'atteindre les cibles physiologiques à court terme et à long terme. Il peut être décidé que dans certains niveaux d'activité du patient, la VNS n'est pas délivrée (état S0), par exemple durant le "sommeil".

Le patient est alors stimulé avec ce jeu de paramètres. Un module d'analyse 253 et le module de reconfiguration des paramètres reçoivent l'état courant CS (définissant le jeu de paramètres courant) et le niveau de la variable physiologique PVL obtenu avec cet état, et déterminent si le modèle de transition d'état doit être reconfiguré ou non.

Une caractéristique avantageuse de cette approche adaptative réside en ce que le module d'analyse et le module de reconfiguration de paramètres sont aptes à détecter les configurations de paramètres (états) provoquant des effets secondaires. Si un état provoque des effets secondaires (cet effet pouvant être détecté comme précédemment par les capteurs disponibles), alors le module de reconfiguration des paramètres rejette cet état (le rend indisponible), ou modifie les valeurs des paramètres VNS de cet état.

Le module de reconfiguration de paramètres 252 adapte le modèle de transition d'état si nécessaire, selon des approches telles qu'on va les décrire dans la suite.

Une autre application de cette approche adaptative peut inclure l'activation de protocoles de stimulation VNS préprogrammés, aboutissant à une mise à jour des réponses attendues pour chaque état ($RS_{Qk,Nk,k}$), soit de façon régulière dans le temps, soit à la survenance de tout événement spécifié, comme on va le décrire dans la suite. La Figure 26 montre l'organigramme de l'approche adaptative proposée.

On va maintenant décrire différentes formes de réalisation de cette approche adaptative.

On rappellera tout d'abord que l'adaptation du modèle de transition d'état est avantageusement réalisée avec différentes résolutions temporelles.

Dans une première forme de réalisation, on réalise une estimation des paramètres déportée dans un système externe 270 comprenant le module de reconfiguration de paramètres 252 et le module d'analyse 253, comme illustré sur la Figure 27. Cette estimation est basée sur un balayage des paramètres et une analyse de ses effets, comme décrit plus haut. Cette estimation peut être réalisée en per- ou post-opératoire lors d'une séance de suivi, en utilisant un module d'analyse déporté.

Dans une deuxième forme de réalisation, illustrée sur la Figure 28, on réalise une estimation adaptative des paramètres de façon intégrée, c'est-à-dire que la mise à jour des valeurs RS et la réorganisation de la matrice de transition T sont réalisées au sein même du dispositif implantable, ce qui implique que le module d'analyse 253 et le module de reconfiguration 252 sont embarqués dans le dispositif implantable.

Ainsi le dispositif implantable est ici auto-adaptatif et réalise de façon autonome un apprentissage des valeurs typiques.

En résumé, la fonctionnalité d'estimation adaptative des paramètres est un raffinement du système de commande à transition d'état décrit dans ce qui précède, qu'il soit mis en oeuvre sur une base, déterministe, stochastique ou sur un ensemble de dispositifs de contrôle combinant approches déterministe et stochastique.

Dans le cas d'une adaptation selon plusieurs résolutions temporelles déportée, avec le mode de réalisation de la Figure 27, alors l'adaptation du modèle de transition d'état peut être réalisée à différentes résolutions temporelles telles que, de façon non limitative, n fois par jour, n fois par semaine, n fois par mois, n fois par année, de façon programmée par le praticien avec un suivi correspondant.

Dans le cas de la forme de réalisation intégrée correspondant à la Figure 28, l'adaptation du modèle de transition d'état peut être réalisée avec différentes résolutions temporelles telles que, de façon non limitative, n fois par heure, n fois par jour, quotidiennement, une fois par semaine, une fois par mois, une fois par an.

En ce sens, la forme de réalisation de la Figure 28 peut être utilisée pour adapter des dispositifs de commande dédiés à la régulation de variables physiologiques sur le court terme (par exemple l'intervalle RR, les pics de variation de pression artérielle $(dP/dt)_{max}$, l'activité physique) et à la régulation de variables physiologiques variables à long terme (par exemple le rythme cardiaque HR, la variabilité du rythme cardiaque HRV).

En outre, le mode de réalisation de la Figure 27 peut être utilisé pour adapter les dispositifs de commande de transition d'état dédiés à la régulation de variables physiologiques sur le long terme.

## Revendications

**1.** Système de traitement par stimulation, comprenant :

- un dispositif de stimulation (28) tel qu'un générateur d'impulsions connecté à une ou plusieurs électrodes aptes à être placées sur ou à proximité d'un nerf du système nerveux autonome ;
- au moins un capteur de signaux physiologiques et/ou physiques et des unités de traitement de ces signaux, capables de fournir au moins un niveau physiologique/physique courant (*PVLcourant*) ; et

- des moyens de commande du dispositif de stimulation selon un jeu de paramètres de stimulation ;

**caractérisé en ce qu'**il comprend au moins un module de contrôle (227) comportant :

- un modèle de transition d'états (2271), ledit modèle comprenant une matrice de transitions (T) et une matrice de connexion (C) ;
les états (Si) dudit modèle étant caractérisés chacun par un jeu de valeurs des paramètres de stimulation (Pi), associé à au moins une réponse physiologique/physique attendue (RSi) lors de l'application d'une stimulation avec ces paramètres ; et
- un dispositif de commande de transition d'états (2272) déterminant de façon organisée les transitions d'un état courant (Si) vers un nouvel état (Sj), provoquant un changement correspondant du jeu de valeurs des paramètres utilisés pour la stimulation, d'un jeu de paramètres courant (Pi) vers un nouveau jeu de paramètres (Pj).

2. Le système de la revendication 1, dans lequel le modèle de transition d'états est de type déterministe, les matrices de transition étant connues à l'initialisation du système.

3. Le système de la revendication 2, dans lequel le dispositif de commande est apte à détecter une situation physique et/ou physiologique anormale et à réaliser une transition vers un état (S0) d'absence de stimulation en réponse à cette détection.

4. Le système de la revendication 2 ou 3, dans lequel le dispositif de commande est apte à déterminer d'éventuelles transitions entre états (Si) de façon régulière et prédéterminée dans le temps.

5. Le système de la revendication 2 ou 3, dans lequel le dispositif de commande est apte à déterminer d'éventuelles transitions entre états (Si) en réponse à la survenue d'un ou plusieurs évènements prédéterminés.

6. Le système de la revendication 2, 3, 4 ou 5, dans lequel le modèle de transition déterministe est séquentiel, les différents états étant ordonnés selon l'effet qu'ils provoquent sur le patient, une transition à partir d'un état ne pouvant être effectuée qu'avec un pas donné vers un état d'ordre supérieur, vers un état d'ordre inférieur, ou vers le même état.

7. Le système de la revendication 6, dans lequel le pas est fixe.

8. Le système de la revendication 6, dans lequel le pas est variable.

9. Le système de la revendication 2, 3, 4 ou 5, dans lequel le modèle de transition déterministe est à base de dichotomie entre états de niveau minimal et de niveau maximal.

10. Le système de la revendication 2, 3, 4 ou 5, dans lequel le modèle de transition déterministe est optimisé, le dispositif de commande déterminant l'état contenant la réponse la plus basse pour laquelle la différence entre le niveau physiologique mesuré et le niveau physiologique cible est inférieure à un seuil donné.

11. Le système de la revendication 1, dans lequel la ou les matrices mémorisées correspondent à un modèle de transition stochastique, chaque cellule contenant une valeur de probabilité (ai,j) de transition d'un état initial (Si) vers un nouvel état (Sj), et la somme des valeurs de probabilités des transitions possibles à partir d'un état donné (i) vers tout nouvel état étant égale à 1.

12. Le système de la revendication 11, dans lequel le modèle de transition d'état est un Modèle de Markov Caché (*Hidden Markov Model*).

13. Le système de la revendication 11 ou 12, dans lequel le dispositif de commande est apte à détecter une situation physique et/ou physiologique anormale et à réaliser une transition vers un état (S0) d'absence de stimulation en réponse à cette détection.

14. Le système de l'une des revendications 1 à 13, dans lequel le au moins un capteur est un capteur apte à être intégré dans un dispositif médical implantable, et apte à délivrer : un signal d'électrogramme cardiaque, musculaire ou nerveux ; un signal d'accélération corporelle, cardiaque ou musculaire ; un signal de bioimpédance respiratoire, de débit cardiaque ou de pression ; un signal de température ; et/ou un signal piézométrique de pression ou de contractilité cardiaque.

**15.** Le système de la revendication 14, dans lequel ledit niveau physiologique et physique (*PVLcourant*) est déterminé à partir de l'une des variables physiologiques ou physiques suivantes : fréquence cardiaque, variabilité du rythme sinusal, pression artérielle, contractilité cardiaque, activité physique, température, mouvements, fréquence respiratoire, ou toute combinaison de ces variables.

**Patentansprüche**

**1.** Stimulationsbehandlungssystem, umfassend:

- eine Stimulationsvorrichtung (28), beispielsweise einen Impulsgenerator, der mit einer oder mehreren Elektroden verbunden ist, die an oder in der Nähe eines Nervs des autonomen Nervensystems angeordnet werden können;
- mindestens eine physiologische und/oder physikalische Signalsensor und Verarbeitungseinheiten für diese Signale, die in der Lage sind, mindestens einen physiologischen/physikalischen Strompegel (PVL-Strom) bereitzustellen; und
- Steuermittel der Stimulationsvorrichtung gemäß einem Satz von Stimulationsparametern;

**dadurch gekennzeichnet, dass** es mindestens ein Steuermodul (227) enthält, umfassend:

- ein Zustandsübergangsmodell (2271), wobei dieses Modell eine Übergangsmatrix (T) und eine Verbindungsmatrix (C) umfasst;
wobei die Zustände (Si) des Modells durch einen Satz von Werten der Stimulationsparameter (Pi) gekennzeichnet und jeweils mit mindestens einer erwarteten physiologischen/physischen Reaktion (RSi) beim Anlegen einer Stimulation mit diesen Parametern verbunden sind; und
- eine Zustandsübergangssteuervorrichtung (2272), die sequentiell die Übergänge von einem aktuellen Zustand (Si) zu einem neuen Zustand (Sj) bestimmt, wodurch eine entsprechende Änderung in dem Satz von Werten der zur Stimulation verwendeten Parameter bewirkt wird, nämlich von von einem aktuellen Satz von Parametern (Pi) zu einem neuen Satz von Parametern (Pj).

**2.** System nach Anspruch 1, wobei das Zustandsübergangsmodell des deterministischen Typ ist, wobei die Übergangsmatrizen bei der Initialisierung des Systems bekannt ist.

**3.** System nach Anspruch 2, wobei die Steuervorrichtung in der Lage ist, eine abnormale physische und/oder physiologische Situation zu erfassen und als Reaktion auf diese Erfassung einen Zustand (SO) ohne Stimulation zu erreichen.

**4.** System nach Anspruch 2 oder 3, wobei die Steuervorrichtung dazu eingerichtet ist, mögliche Übergänge zwischen Zuständen (Si) regelmäßig und zeitlich vorbestimmt zu erfassen.

**5.** System nach Anspruch 2 oder 3, wobei die Steuervorrichtung dazu eingerichtet ist, mögliche Übergänge zwischen Zuständen (Si) als Reaktion auf das Auftreten eines oder mehrerer vorbestimmter Ereignisse zu erfassen.

**6.** System nach Anspruch 2, 3, 4 oder 5, wobei das deterministische Übergangsmodell sequentiell ist, wobei die verschiedenen Zustände nach der Wirkung angeordnet sind, die sie auf den Patienten verursachen, während ein Übergang von einem Zustand nur mit einer gegebenen Steigung zu einem Zustand höherer Steigung, einen Zustand niedrigerer Steigung oder den gleichen Zustand erfolgen kann.

**7.** System nach Anspruch 6 , wobei die Steigung fest ist.

**8.** System nach Anspruch 6 , wobei die Steigung variabel ist.

**9.** System nach Anspruch 2, 3, 4 oder 5, wobei das deterministische Übergangsmodell auf einer Dichotomie zwischen Zuständen mit mittlerem und maximalem Pegel basiert ist.

**10.** System nach Anspruch 2, 3, 4 oder 5, wobei das deterministische Übergangsmodell optimisiert ist, wobei der Kontroller den Zustand mit der niedrigsten Reaktion bestimmt, für die die Differenz zwischen dem gemessenen physiologischen Pegel und dem physiologischen Soll-Pegel unterhalb einer Schwelle gegebenen ist.

11. System nach Anspruch 1, wobei die gespeicherte(n) Matrix oder Matrizen einem stochastischen Übergangsmodell entsprechen, wobei jede Zelle einen Übergangswahrscheinlichkeitswert (ai, j) von einem Anfangszustand (Si) zu einem neuen Zustand (Sj) enthält, und die Summe der Wahrscheinlichkeitswerte der möglichen Übergänge von einem gegebenen Zustand (i) bis zu einem neuen Zustand gleich 1 ist.

12. System nach Anspruch 11, wobei das Zustandsübergangsmodell ein Hidden-Markov-Modus 1 ist.

13. System nach Anspruch 11 oder 12, wobei die Steuervorrichtung dazu eingerichtet ist, eine abnormale physische und/oder physiologische Situation zu erkennen und eine Übergang als Reaktion auf einen Zustand (SO) ohne Stimulation zu verursachen.

14. System nach einem der Ansprüche 1 bis 13, wobei der mindestens eine Sensor angepasst ist, in einer implantierbaren medizinischen Vorrichtung integriert zu werden, und in der Lage ist, folgendes zu liefern: ein Herz-, Muskel- oder Nervenelektrogrammsignal; ein Körper-, Herz- oder Muskelbeschleunigungssignal; ein Atmungs-Bioimpedanz-, Herzausstoß- oder Drucksignal; ein Temperatursignal; und/oder ein piezometrischen Druck- oder Herzkontraktilitätssignal.

15. System nach Anspruch 14, wobei dieser physiologische und physikalische Pegel (PVL-Strom) aus einer der folgenden physiologischen oder physiologischen Variablen bestimmt wird: Herzfrequenz, Sinusrhythmusvariabilität, Blutdruck, Herzkontraktilität, körperlicher Aktivität, Temperatur, Bewegung, Atemfrequenz oder aus einer Kombination dieser Variablen.

**Claims**

1. A stimulation therapy system, comprising:

   - a stimulation device (28) such as a pulse generator connected to one or more electrodes adapted to be placed on or near a nerve of the autonomic nervous system;
   - at least one physiological and/or physical signal sensor and processing units for these signals, capable of providing at least one physiological/physical current level (PVLcurrent); and
   - control means of the stimulation device according to a set of stimulation parameters;

   **characterised in that** it comprises at least one control module (227) comprising:

   - a state transition model (2271), said model comprising a transition matrix (T) and a connection matrix (C); the states (Si) of said model being each **characterised by** a set of values of the stimulation parameters (Pi), associated with at least one expected physiological/ physical response (RSi) when applying a stimulation with these parameters; and
   - a state transition control device (2272) sequentially determining the transitions from a current state (Si) to a new state (Sj), causing a corresponding change in the set of values of the parameters used for stimulation, from a current set of parameters (Pi) to a new set of parameters (Pj).

2. The system of claim 1, wherein the state transition model is of the deterministic type, the transition matrices being known at the initialisation of the system.

3. The system of claim 2, wherein the control device is able to detect an abnormal physical and/or physiological situation and to make a transition to a state (SO) of no stimulation in response to this detection.

4. The system of claim 2 or 3, wherein the control device is adapted to determine possible transitions between states (Si) regularly and predetermined in time.

5. The system of claim 2 or 3, wherein the control device is adapted to determine possible transitions between states (Si) in response to the occurrence of one or more predetermined events.

**6.** The system of claim 2, 3, 4 or 5, wherein the deterministic transition model is sequential, the different states being ordered according to the effect they cause on the patient, whereas a transition from a state can only be performed with a given pitch to a higher order state, to a lower order state, or to the same state.

**7.** The system of claim 6, wherein the pitch is fixed.

**8.** The system of claim 6, wherein the pitch is variable.

**9.** The system of claim 2, 3, 4 or 5, wherein the deterministic transition model is based on a dichotomy between mid-level and maximum level states.

**10.** The system of claim 2, 3, 4 or 5, wherein the deterministic transition model is optimised, wherein the controller determines the state containing the lowest response for which the difference between the measured physiological level and the target physiological level is below a given threshold.

**11.** The system of claim 1, wherein the stored matrix or matrices correspond to a stochastic transition model, each cell containing a transition probability value $(a_{i,j})$ from an initial state $(S_i)$ to a new state $(S_j)$, and the sum of the probabilities values of the possible transitions from a given state $(i)$ to any new state being equal to 1.

**12.** The system of claim 11, wherein the state transition model is a Hidden Markov Model.

**13.** The system of claim 11 or 12, wherein the control device is adapted to detect an abnormal physical and/or physiological situation and to make a transition to a state (SO) of no stimulation in response to this detection.

**14.** The system of one of claims 1 to 13, wherein said at least one sensor is a sensor adapted to be integrated into an implantable medical device, and capable of delivering:

a cardiac, muscular or nervous electrogram signal; a body, cardiac or muscular acceleration signal; a respiratory bioimpedance, cardiac output or pressure signal;
a temperature signal; and/or a piezometric pressure or cardiac contractility signal.

**15.** The system of claim 14, wherein said physiological and physical level (PVLcurrent) is determined from one of the following physiological or physiological variables: heart rate, sinus rhythm variability, blood pressure, cardiac contractility, physical activity, temperature, movement, respiratory rate, or any combination of these variables.

11

$s_1$

$s_n$

21

29

40

30

50

22 23

80

24 26

25

70

60

12 28 27

**Fig.1**

10 20

20

225

PHYSIOLOGIE

21 221 224

M1

M2

223

227a 227n

2271 2272

28

229

S2

S1 S3

SN S4

226

230

**Fig.2**

**Fig.3**

227

2271

2272

225

Niveau
PVL courant

226

Nouvel état CS

**Fig.4**

A

Ad

As

Ads

Abd

Ao

Adspf

Adspv

**Fig.5**

$S_0$    $S_1$    $S_2$    . . .    $S_{N-1}$    $S_N$

# Fig.6

2272

225

PVL COURANT

INTERFACE

226

PVL CIBLE

CONDITION
SATISFAITE?

NON

OUI

Compteur
CT

OUI

CT > d

CT > d

OUI

NON

NON

CS=CS+1
CT=0

CS=CS
CT=CT+1

CS=CS-1
CT=0

Nouvel état CS

28

# Fig.7

Intervalle RR (ms)

510

500

490

480

470

460

450

440

CS=11   CS=11   CS=11

CS=10   CS=10   CS=10   CS=10

CS=9

CS=8

CS=7

CS=6

CS=5

d=5   CS=4

d=5   CS=3

d=5   CS=2

d=5   CS=1

d=5

CS=0

——— Intervalle RR instantané
- - - Cible

10   20   30   40   50   60   70   80   90

Nombre de battements

# Fig.8

# Fig.9

## Fig.10

101

225

$CS = \dfrac{CS_{max} - CS_{min}}{2}$

226

102

NON

OUI

$CS_{min} = CS$

$CS = \dfrac{CS_{max} - CS_{min}}{2}$

$CS_{max} = CS$

$CS = \dfrac{CS_{max} - CS_{min}}{2}$

103

104

CS

Nouvel état CS

28

## Fig.10bis

225

Init

105

OUI

NON

CS=Sk

Nouvel état CS

28

**a)**

**Fig.11**

0,8

0,55 (S₁) (S₂) 0,2

0,45

**b)**

0,4

0,4 (S₂) 0,1 (S₂) 0,75

0,2

0,8

0,1 0,15

(S₂)

0,3

**c)**

0,65

0,25 (S₁) (S₂) 0,2

0,8

Intervalle RR cible

**Fig.12**

Intervalle RR instantané

500
450
PVL 400

5
4
3
Etat courant (CS) 2
1

1    2    3  ·······

Evènement

**Fig.13**

**Fig.14**

**Fig.15**

**Fig.16**

**Fig.17**

**Fig.18**

Stimulation avec
paramètres $P_i$

| PATIENT 1 | PATIENT 2 | PATIENT N |
|---|---|---|
| Réponse PVL1 | Réponse PVL2 | Réponse PVL3 |

Effets
indésirables
?

OUI → $i = i+1$

NON

Base de données de population

Analyse des données

Architecture -
Paramètres -
Matrices C et T

**Fig.19**

Apprentissage
spécifique
au patient

Architecture -
Paramètres -
Matrices C et T

EP 3 113 033 B1

## Fig.20

```
┌──────────────────┐
│  Base de données │
│  de population   │
└──────────────────┘
          │
          ▼
┌──────────────────┐
│     Analyse      │
│   des données    │
└──────────────────┘
          │
          ▼
┌──────────────────┐        ┌──────────────────┐
│   Paramètres     │───────▶│  Apprentissage   │
│   initialisés    │        │   spécifique     │
└──────────────────┘        │   du patient     │
                            └──────────────────┘
                                     │
                                     ▼
                            ┌──────────────────┐
                            │  Architecture -  │
                            │  Paramètres -    │
                            │  Matrices C et T │
                            └──────────────────┘
```

## Fig.21

# Fig.22

| | N | | Période | | Amplitude |
|---|---|---|---|---|---|
| P0,0 | 0 | P0,1 | 0 | P0,2 | 0 |
| P1,0 | 1 | P1,1 | 22 | P1,2 | 1 |
| P2,0 | 2 | P2,1 | 12 | P2,2 | 1 |
| P3,0 | 2 | P3,1 | 34 | P3,2 | 1 |
| P4,0 | 2 | P4,1 | 21 | P4,2 | 1 |
| P5,0 | 3 | P5,1 | 22 | P5,2 | 1 |
| P6,0 | 3 | P6,1 | 23 | P6,2 | 1 |
| P7,0 | 3 | P7,1 | 38 | P7,2 | 1 |
| P8,0 | 4 | P8,1 | 22 | P8,2 | 1 |
| P9,0 | 4 | P9,1 | 30 | P9,2 | 1 |
| P10,0 | 4 | P10,1 | 38 | P10,2 | 1 |
| P11,0 | 1 | P11,1 | 22 | P11,2 | 2 |
| P12,0 | 2 | P12,1 | 22 | P12,2 | 2 |
| P13,0 | 2 | P13,1 | 30 | P13,2 | 2 |
| P14,0 | 2 | P14,1 | 38 | P14,2 | 2 |
| P15,0 | 3 | P15,1 | 22 | P15,2 | 2 |
| P16,0 | 3 | P16,1 | 30 | P16,2 | 2 |
| P17,0 | 3 | P17,1 | 30 | P17,2 | 2 |
| P18,0 | 4 | P18,1 | 22 | P18,2 | 2 |
| P19,0 | 4 | P19,1 | 30 | P19,2 | 2 |
| P20,0 | 4 | P20,1 | 30 | P20,2 | 2 |
| P21,0 | 1 | P21,1 | 22 | P21,2 | 3 |
| P22,0 | 2 | P22,1 | 22 | P22,2 | 3 |
| P23,0 | 2 | P23,1 | 30 | P23,2 | 3 |
| P24,0 | 2 | P24,1 | 38 | P24,2 | 3 |
| P25,0 | 3 | P25,1 | 22 | P25,2 | 3 |
| P26,0 | 3 | P26,1 | 30 | P26,2 | 3 |
| P27,0 | 3 | P27,1 | 38 | P27,2 | 3 |
| P28,0 | 4 | P28,1 | 22 | P28,2 | 3 |
| P29,0 | 4 | P29,1 | 30 | P29,2 | 3 |
| P30,0 | 4 | P30,1 | 38 | P30,2 | 3 |

# Fig.23

# Fig.24

# Fig.25

PHYSIOLOGIE

21

PV

225

226

INTERFACE

RS(CS)

RS(CS)

253

252

CS

CS

RS(CS)

227

PVL
court-terme

PVL
long terme

227₁

227₂

251

229

28

# Fig.26

21

225

PVL calculé

Niveau PVL
attendu (RS)

2271

[RS-PVL]
>
SEUIL
PVL_CIBLE

NON

Pas de reconfiguration
- pas de diagnostic

OUI

253

252

Paramètres
reconfigurés

2271

# Fig.27

# Fig.28

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2014288620 A1
- US 2006293720 A1
- US 2005240242 A1
- US 7783349 B2
- US 7509166 B2
- US 2012245656 A1
- WO 2011137029 A
- EP 1102607 A1

**Littérature non-brevet citée dans la description**

- **A. AARABI ; H. BIN.** Seizure prédiction in intracranial EEG: A patient-specific rule-based approach. *Engineering in Medicine and Biology Society, EMBC, 2011 Annual International Conference of the IEEE,* 2566-2569
- **I. CAPEL ; M. RIGLA ; G. GARCÍA-SÁEZ ; A. RODRIGUEZ-HERRERO ; B. PONS ; D. SUBÍAS ; F. GARCÍA-GARCÍA ; M. GALLACH ; M. AGUILAR ; C. PÉREZ-GANDÍA.** Artificial Pancreas Using a Personalized Rule-Based Controller Achieves Overnight Normoglycemia in Patients with Type 1 Diabetes. *Diabetes Technol Ther.,* 2014, vol. 16 (3), 172-179
- **F. PORÉE ; A. KACHENOURA ; G. CARRAULT ; R. D. MOLIN ; P. MABO ; A. I. HERNÁNDEZ.** Surface Electrocardiogram Reconstruction From Intra-cardiac Electrograms Using a Dynamic Time Delay Artificial Neural Network. *Biomedical Engineering, IEEE Transactions on,* 2013, vol. 60, 106-114
- **H. M. ROMERO UGALDE ; J.-C. CARMONA ; V. M. ALVARADO ; J. REYES-REYES.** Neural Network Design and Model Reduction Approach for Black Box Non Linear System Identification with Reduced Number of Parameters. *Neurocomputing,* 2013, vol. 101, 170-180
- **M. LÔHNING ; M. REBLE ; J. HASENAUER ; S. YU ; F. ALLGÖWER.** Model predictive control using reduced order models: Guaranteed stability for constrained linear systems. *Journal of Process Control,* 2014, vol. 24 (11), 1647-1659